# EUROPEAN PATENT APPLICATION

(11) **EP 3 121 746 A1**
(43) Date of publication of application: **25.01.2017**
(21) Application number: 16170931.6
(22) Date of filing: 23.05.2016
(51) Int. Cl.: G06F 19/00

(54) **INFORMATION ANALYSIS ASSISTANCE DEVICE, OPERATION METHOD AND OPERATION PROGRAM THEREOF, AND INFORMATION ANALYSIS ASSITANCE SYSTEM**

(30) Priority: 21.07.2015 JP 2015143654
(71) Applicant: Fujifilm Corporation, Minato-ku Tokyo 106-8620 (JP)
(72) Inventor: OKABE, Yuuki, Tokyo, 107-0052 (JP); KAMODA, Rena, Tokyo, 107-0052 (JP)
(74) Representative: Klunker . Schmitt-Nilson . Hirsch

(57) **Abstract**

There are provided an information analysis assistance device, an operation method and operation program thereof, and an information analysis assistance system capable of understanding information from many angles. In a case where a date in a table is selected by a first selection method, a screen generation unit sets a first display format to display windows showing the outline of each piece of medical data side by side in a second display region. In a case where an item is selected by a second selection method, the screen generation unit sets a second display format to display a time-series change table showing time-series changes in medical data in the second display region. In a case where a cell is selected by a third selection method, the screen generation unit sets a third display format to display the details of medical data in the second display region.

## Description

### BACKGROUND OF THE INVENTION

### 1. Field of the Invention

The present invention relates to an information analysis assistance device, an operation method and operation program thereof, and an information analysis assistance system.

### 2. Description of the Related Art

In order to assist the analysis of information, for example, analysis of medical data acquired in a medical procedure of one patient, in techniques disclosed in JP2014-153920A and WO2011/122402A, the history of examination results of medical examinations of a plurality of items performed on one patient is displayed in a table in which a time axis including a date on which a medical examination was performed is assigned as an axis in the row direction and items of medical examinations are assigned as an axis in the column direction. An icon is disposed in a cell that is an intersection between the date and the item in the table, so that it can be seen at a glance on which day what kind of medical examination has been performed. In the techniques disclosed in JP2014-153920A and WO2011/122402A, in order to eliminate the complicatedness of switching operation of the screen, a first display region where a table is displayed and a second display region where examination results of medical examinations are displayed are disposed on the same screen.

JP2014-153920A discloses a display format to display the details (for example, an examination image in a case where a medical examination is an image examination) of a medical examination corresponding to a selected icon in a second display region in response to the selection of an icon. WO2011/122402A discloses a display format to display examination results of the latest medical examinations on a date at an arbitrary position in a first display region side by side in a second display region when a mouse is right-clicked at the arbitrary position in the first display region and "display the latest examination at this point in time" is selected. Thus, in the techniques disclosed in JP2014-153920A and WO2011/122402A, a selection operation on the table serves as a trigger to display the examination results in the second display region.

### SUMMARY OF THE INVENTION

In the case of making any determination by analyzing information, in order to make an accurate determination according to the situation, it is important to understand the information from many angles, that is, from various points of view rather than a one-sided point of view. In particular, in a case where a doctor makes a diagnosis by analyzing the examination results of medical examinations, the point of view of the analysis of the examination results changes with the medical phase, such as with more extensive examination results from the first visit before admission, more detailed examination results immediately before and after surgery, and time-series changes in examination results in the follow-up as an outpatient after surgery. Therefore, there is a great need to understand the information from many angles.

In addition, even in the same medical phase, it is possible to understand information from many angles. For example, when making a diagnosis, an entire image may be taken in more extensive examination results and an item of interest may be checked in more detailed examination results, or time-series changes in the examination results may be checked in order to determine medication.

However, in the techniques disclosed in JP2014-153920A and WO2011/122402A, examination results can be displayed only in the display format of one point of view. For this reason, it was not possible to understand information from many angles in the techniques disclosed in JP2014-153920A and WO2011/122402A.

It is an object of the invention to provide an information analysis assistance device, an operation method and operation program thereof, and an information analysis assistance system capable of understanding information from many angles.

In order to achieve the aforementioned object, an information analysis assistance device of the invention comprises: a screen generation unit that generates an information display screen having a first display region to display a table, in which two kinds of attributes for classifying information are assigned to an axis in a row direction or an axis in a column direction, and a second display region to display the information; and a screen output control unit that controls an output of the information display screen. The screen generation unit displays the information in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attribute is selected.

It is preferable that the information is medical data of a plurality of items acquired in a medical procedure of one patient. In this case, preferably, the two kinds of attributes are a date on which the medical data has been acquired and an item of the medical data, and the screen generation unit assigns the date to one of the axis in the row direction and the axis in the column direction and assigns the item to the other one. Preferably, in a case where the date is selected by one of the first and second methods, the screen generation unit sets the display format to a first display format in which the medical data items acquired on a selected date are displayed side by side in the second display region. Preferably, in a case where the item is selected by the other one of the first and second methods, the screen generation unit sets the display format to a second display format in which time-series changes in the medical data are displayed in the second display region. Preferably, in a case where the cell is selected by the third method, the screen generation unit sets the display format to a third display format in which details of the medical data are displayed in the second display region.

Preferably, in the first display format, in a case where the medical data of the second display region is selected, the screen generation unit displays details of the selected medical data in the second display region in the same manner as in the third display format.

Preferably, in the first selection method or the second selection method, a plurality of the items are selectable. Preferably, in a case where a plurality of the items are selected, the screen generation unit displays time-series changes in the plurality of pieces of selected medical data side by side in the second display region in the second display format.

Preferably, in the third selection method, a plurality of the cells are selectable. Preferably, in a case where a plurality of the cells are selected, the screen generation unit displays details of the medical data corresponding to the plurality of selected cells side by side in the second display region in the third display format.

Preferably, in the first display format, in a case where there is no medical data acquired on the date selected by the first selection method or the second selection method, the screen generation unit displays the latest medical data of the medical data acquired before the selected date in the second display region. In this case, it is preferable that the screen generation unit displays the medical data acquired on the date selected by the first selection method or the second selection method so as to be distinguished from the other pieces of medical data.

Preferably, the screen generation unit assigns at least a medical examination performed on the patient and a drug administered to the patient, as the items, to one of the axis in the row direction and the axis in the column direction, and the screen generation unit displays an examination result of the medical examination, the drug, and a dose in the second display region as the medical data.

It is preferable that the examination result has a plurality of examination items. In this case, preferably, in the first display format, in a case where the examination result acquired during a first visit is displayed in the second display region, the screen generation unit performs display so as to include examination items other than main examination items set in advance of the plurality of examination items. Preferably, in a case where the examination result acquired at a time other than the first visit is displayed in the second display region, the screen generation unit displays only the main examination items.

Preferably, in the first display format, the screen generation unit increases or decreases the number of examination items of one examination result to be displayed in the second display region according to the number of examination results to be displayed side by side in the second display region.

Preferably, in the first display format, the screen generation unit popup-displays details of the medical data in the second display region. In this case, it is preferable that the screen generation unit displays the cell corresponding to the medical data of which details are popup-displayed so as to be distinguished from the other cells.

Preferably, in the first display format, the screen generation unit further displays a medical record description, an event including admission and discharge, and history in the second display region as the medical data.

An operation method of an information analysis assistance device of the invention comprises: a screen generation step of generating an information display screen having a first display region to display a table, in which two kinds of attributes for classifying information are assigned to an axis in a row direction or an axis in a column direction, and a second display region to display the information; and a screen output control step of controlling an output of the information display screen. In the screen generation step, the information is displayed in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attributes is selected.

An operation program of an information analysis assistance device of the invention causes a computer to execute: a screen generation function of generating an information display screen having a first display region to display a table, in which two kinds of attributes for classifying information are assigned to an axis in a row direction or an axis in a column direction, and a second display region to display the information; and a screen output control function of controlling an output of the information display screen. By the screen generation function, the information is displayed in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attributes is selected.

An information analysis assistance system of the invention is an information analysis assistance system comprising an information analysis assistance device and a client terminal connected to the information analysis assistance device through a network. The information analysis assistance system comprises: a screen generation unit that generates an information display screen having a first display region to display a table, in which two kinds of attributes for classifying information are assigned to an axis in a row direction or an axis in a column direction, and a second display region to display the information; and a screen output control unit that controls an output of the information display screen. The screen generation unit displays the information in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attributes is selected.

According to the invention, in the information display screen having a first display region to display a table, in which two kinds of attributes for classifying information are assigned to the axis in the row direction or the axis in the column direction, and a second display region to display the information, the information is displayed in the second display region in three different display formats according to each of selection methods of the first selection method in which the attributes assigned to the axis in the row direction are selected, the second selection method in which the attributes assigned to the axis in the column direction are selected, and the third selection method in which a cell that is an intersection between the two kinds of attributes is selected. Therefore, it is possible to provide the information analysis assistance device, the operation method and operation program thereof, and the information analysis assistance system capable of understanding information from many angles.

### BRIEF DESCRIPTION OF THE DRAWINGS

Fig. 1 is a diagram showing an information analysis assistance system.
Fig. 2 is a diagram showing the content of electronic medical records stored in a medical record DB.
Fig. 3 is a diagram showing the content of an examination image stored in an image DB.
Fig. 4 is a diagram showing the content of a medical report stored in a report DB.
Fig. 5 is a block diagram showing a computer that forms a client terminal and an information analysis assistance server.
Fig. 6 is a block diagram showing each functional unit of a CPU of each of a client terminal and an information analysis assistance server.
Fig. 7 is a diagram showing a patient ID input screen.
Fig. 8 is a diagram showing an information display screen in a case where a date is selected.
Fig. 9 is a diagram showing a first display region.
Fig. 10 is a diagram showing a second display region in a case where a date is selected.
Fig. 11 is an explanatory diagram showing to which date the examination result of a medical examination displayed in a window of the second display region corresponds.
Fig. 12 is a diagram showing an information display screen in a case where an item is selected.
Fig. 13 is a diagram showing a second display region in a case where an item is selected.
Fig. 14 is a diagram showing an information display screen in a case where a plurality of items are selected.
Fig. 15 is a diagram showing an information display screen in a case where a cell is selected.
Fig. 16 is a diagram showing a second display region in a case where a cell is selected.
Fig. 17 is a diagram showing an information display screen in a case where a plurality of cells are selected.
Fig. 18 is a diagram showing another example of the information display screen in a case where a cell is selected.
Fig. 19 is a diagram showing another example of the information display screen in a case where a plurality of cells are selected.
Fig. 20 is an explanatory diagram showing the relationship between a selection method of a table and a display format of an information display screen.
Fig. 21 is an explanatory diagram showing the relationship between a selection method of a table and a display format of an information display screen.
Fig. 22 is a flowchart showing the procedure of a CPU of an information analysis assistance server.
Fig. 23 is an explanatory diagram showing a second embodiment.
Fig. 24 is an explanatory diagram showing a third embodiment.
Fig. 25 is an explanatory diagram showing a fourth embodiment.
Fig. 26 is an explanatory diagram showing a fifth embodiment.

### DESCRIPTION OF THE PREFERRED EMBODIMENTS

### [First embodiment]

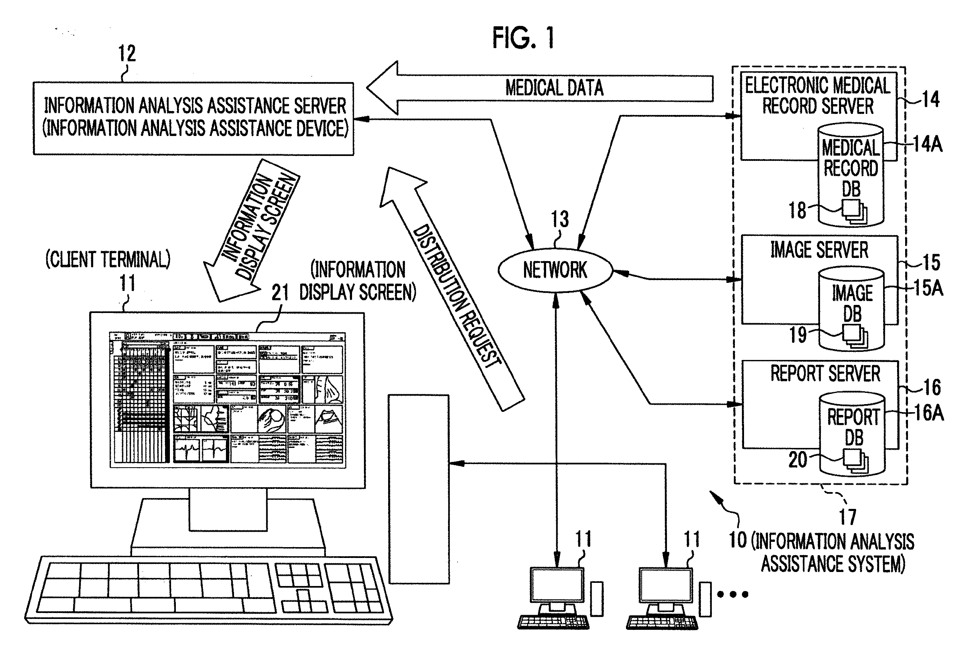

In Fig. 1, an information analysis assistance system 10 is constructed in a medical facility, and comprises a client terminal 11, an information analysis assistance server 12 corresponding to an information analysis assistance device, and the like. The client terminal 11 and the information analysis assistance server 12 are communicably connected to each other through a network 13, such as a local area network (LAN) provided in the medical facility.

An electronic medical record server 14, an image server 15, and a report server 16 (hereinafter, referred to collectively as a server group 17) are connected to the network 13. The electronic medical record server 14 has a medical record database (hereinafter, abbreviated as a database (DB)) 14A, and the electronic medical record 18 is searchably recorded in the medical record DB 14A. The image server 15 has an image DB 15A, and the examination image 19 obtained by various image examinations is searchably recorded in the image DB 15A. The report server 16 has a report DB 16A, and a medical report 20 in which findings, which are the result of interpretation of the examination image 19 by a radiologist, are summarized, is searchably recorded in the report DB 16A.

Each of the client terminal 11, the information analysis assistance server 12, and the server group 17 is configured by installing a control program, such as an operating system, or various application programs on a computer as a base, such as a personal computer, a server computer, or a workstation.

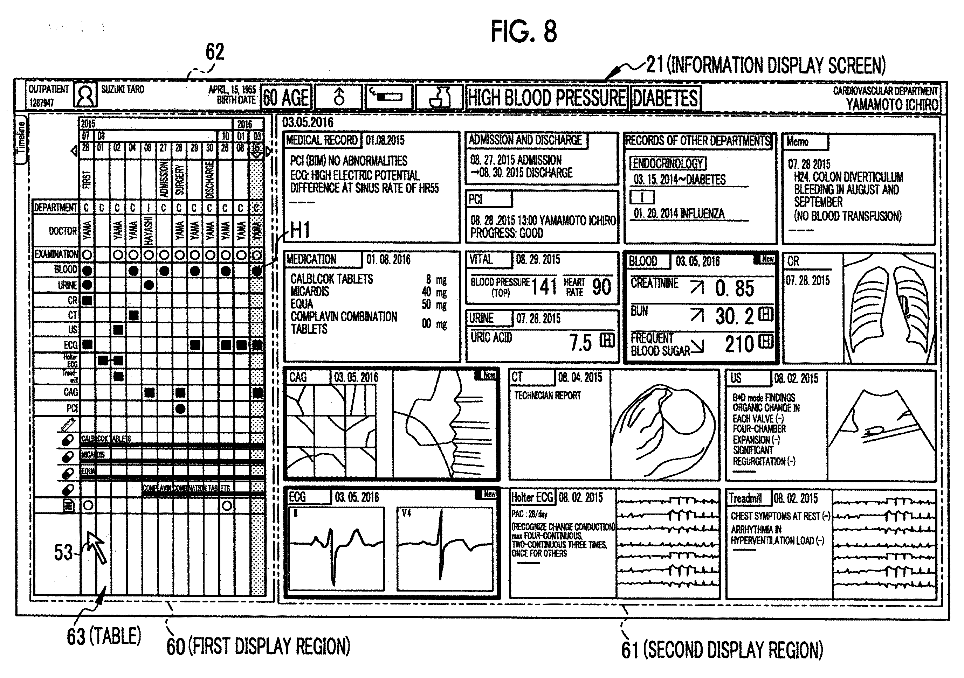

The information analysis assistance server 12 has a function of outputting an information display screen 21 (refer to Fig. 8 or the like) for assisting the analysis of medical data, which has been obtained in the course of medical treatment for one patient in a medical facility, as information. The server group 17 has a function of managing the electronic medical record 18, the examination image 19, and the medical report 20.

The client terminal 11 is operated by a doctor who performs medical treatment for a patient, a nurse who nurses a patient, and a laboratory technician or the like who performs various medical examinations (hereinafter, referred to collectively as a medical staff). A plurality of client terminals 11 are installed for each department, such as internal medicine, surgery, examination department, and rehabilitation department, or for each medical staff. The client terminal 11 is used when performing medical treatment for a patient using various functions of the information analysis assistance server 12 and the server group 17. Specifically, the client terminal 11 is used when viewing the electronic medical record 18, the examination image 19, the medical report 20, or the information display screen 21, or when inputting various kinds of medical data in the electronic medical record 18, or when creating the medical report 20. The client terminal 11 may be a stationary type terminal that is placed in each department, or may be a portable terminal carried by each medical staff.

The information analysis assistance server 12 receives a distribution request from the client terminal 11. The information analysis assistance server 12 acquires medical data corresponding to the distribution request from the server group 17, and generates the information display screen 21 based on the acquired medical data. The information analysis assistance server 12 transmits the generated information display screen 21 to the client terminal 11 that is a source of the distribution request.

The information analysis assistance server 12 distributes the information display screen 21 that can be viewed on the web browser. The information analysis assistance server 12 issues an authentication key to the client terminal 11 to allow access to the information analysis assistance server 12. The information display screen 21 distributed from the information analysis assistance server 12 is displayed on the client terminal 11.

The information analysis assistance server 12 distributes the information display screen 21, for example, in the form of image data for web distribution that is created by a markup language, such as Extensible Markup Language (XML). The client terminal 11 reproduces and displays the information display screen 21 on the web browser based on the image data. Instead of the XML, other data description languages, such as JAVASCRIPT (registered trademark) Object Notation (JSON), may be used.

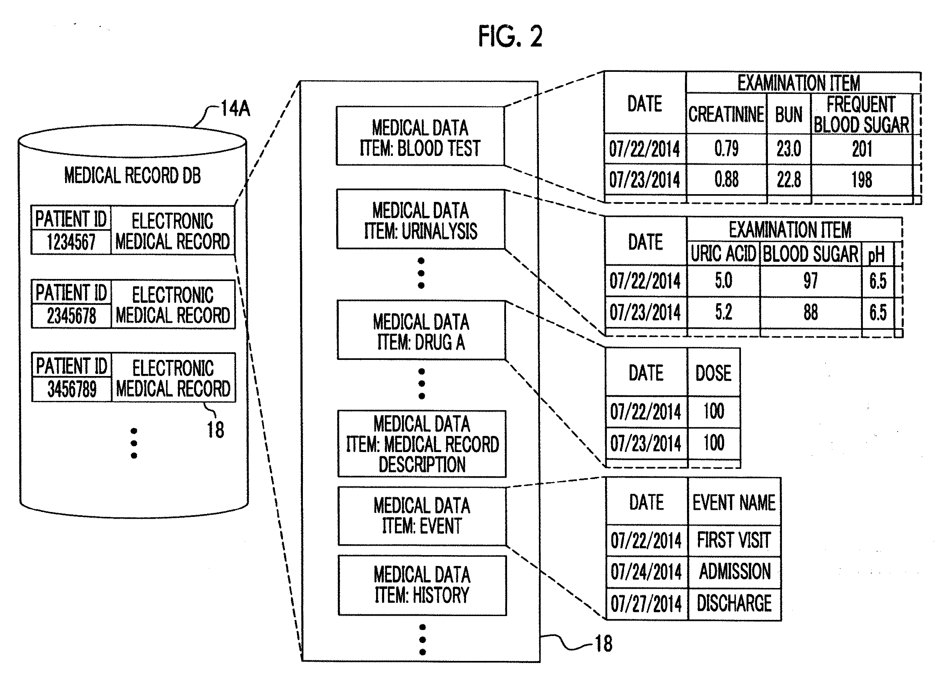

In Fig. 2, the electronic medical record 18 of medical record DB 14A is managed in units of a patient by being associated with a patient identification data (ID), such as "1234567". The electronic medical record server 14 can find the electronic medical record 18 from the medical record DB 14A by using the patient ID as a search key.

The electronic medical record 18 is configured to include medical data of a plurality of items. Items of medical data include various subject examinations performed on a patient, such as a blood test and urinalysis, drugs administered to the patient, medical record descriptions such as a disease name or the content of a medical examination including the main complaint of the patient that is input to the electronic medical record 18 by the doctor, an event that occurs in the course of medical treatment for the patient, such as a first visit and admission and discharge, and a history. Medical data is organized for each of the items, and is recorded in time series.

The record of each item of medical data for one case includes a date, such as a subject examination date, a drug administration date, and an event occurrence date, and the specific content, such as examination values (examination results) of a plurality of examination items of a subject examination, a dose of medicine, and an event name. For example, in a case where the item of medical data is a blood test, the examination items are creatinine, blood urea nitrogen (BUN), frequent blood sugar, and the like. In a case where the item of medical data is urinalysis, the examination items are uric acid, blood sugar, pH (potential hydrogen; hydrogen ion concentration index), and the like.

In addition to the patient ID, patient information, such as patient's name, sex, age, date of birth, and taste (smoking or drinking), is recorded in the electronic medical record 18. In addition to those described above, the items of medical data include vital signs such as the heart rate, pulse, blood pressure, and body temperature of the patient, orders in which instructions of medical examinations, reporting, surgery, medication, and the like are described, and notes such as informative matters between medical staffs or memorandums are described.

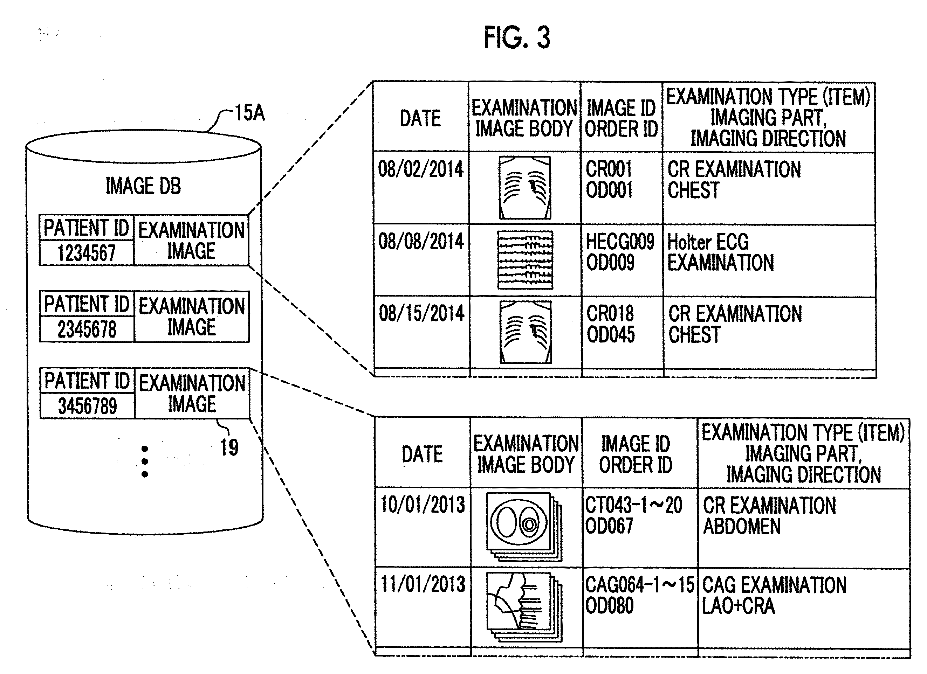

In Fig. 3, the examination image 19 of image DB 15A is managed in units of a patient by being associated with a patient ID, similar to the electronic medical record 18. Similar to the electronic medical record server 14, the image server 15 can find the examination image 19 from the image DB 15A by using the patient ID as a search key.

The examination image 19 is an image obtained by various image examinations. The various image examinations include, for example, an electrocardiogram (ECG) examination (including a Holter ECG examination and a load ECG examination), a coronary angiography (CAG) examination, an ultrasonography (ultrasonic; US) examination, a computed radiography (computer X-ray imaging; CR) examination, a CT (computed tomography) examination, a magnetic resonance imaging (nuclear magnetic resonance imaging; MRI) examination, and an endoscopic examination. The examination image 19 of the CR examination, the CT examination, or the MRI examination is generated in the data file format of digital imaging and communications in medicine (DICOM) specification, for example.

The file of the examination image 19 for one case includes the main body of the examination image 19 and various kinds of accessory information including an image examination date, an image ID and an order ID, types of image examinations such as "CR examination" and "CAG examination", and an imaging part and an imaging direction such as "chest" or "left anterior oblique view (LAO) + cranial (CRA)". The type of image examination is treated as an item of medical data. The image server 15 transmits the examination image 19 and such accessory information to the information analysis assistance server 12 as medical data.

The image ID is a number or symbol for identifying each examination image 19, and the order ID is a number or symbol for identifying an order for instructing various kinds of image examinations. In the case of an image examination in which a plurality of examination images 19 are captured at a time, such as a CT examination or a CAG examination, a common order ID is assigned to each examination image 19 in order to indicate that a plurality of examination images 19 have been obtained by one image examination, and the plurality of examination images 19 are collectively managed as one examination image 19.

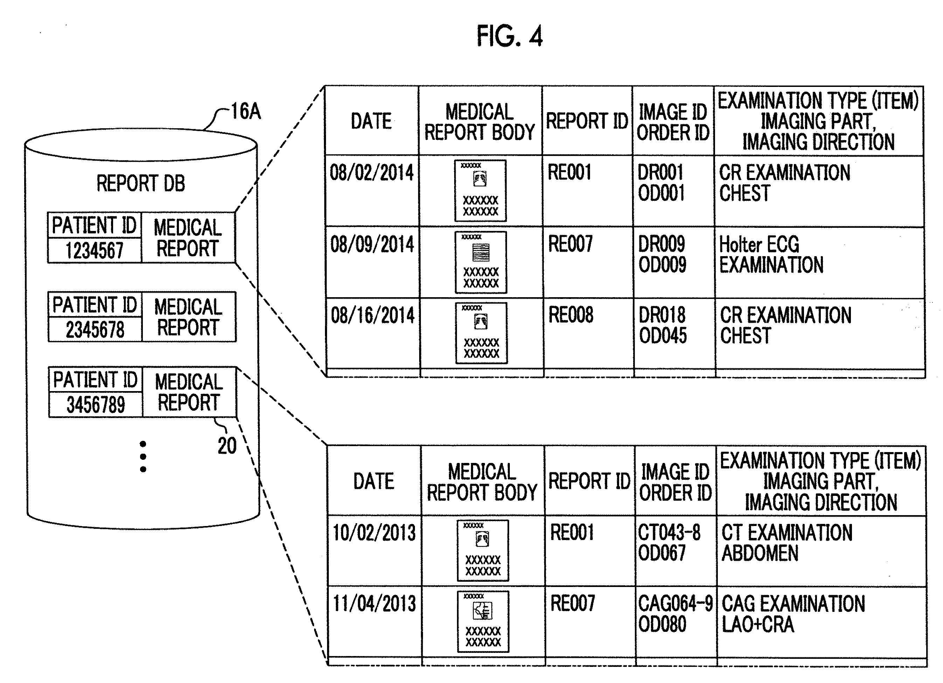

In Fig. 4, the medical report 20 of the report DB 16A is managed in units of a patient by being associated with a patient ID, similar to the electronic medical record 18 and the examination image 19. Similar to the servers 14 and 15, the report server 16 can find the medical report 20 from the report DB 16A by using the patient ID as a search key.

The file of the medical report 20 for one case includes the main body of the medical report 20 and various kinds of accessory information including the creation date of the medical report 20, a report ID, an image ID and an order ID of the examination image 19 attached to the medical report 20, types of image examinations, and an imaging part and an imaging direction. The report server 16 transmits the medical report 20 and such accessory information to the information analysis assistance server 12 as medical data.

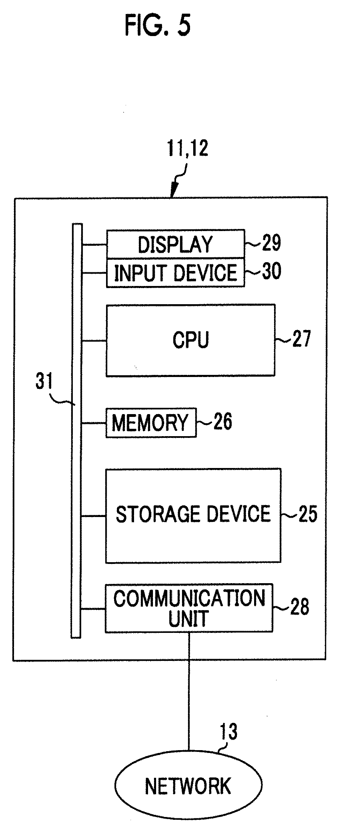

In Fig. 5, the basic configurations of computers that form the client terminal 11 and the information analysis assistance server 12 are the same, and each computer comprises a storage device 25, a memory 26, a central processing unit (CPU) 27, a communication unit 28, a display 29, and an input device 30. These are connected to each other through a data bus 31.

The storage device 25 is a hard disk drive, which is built into a computer that forms the client terminal 11 or the like or which is connected to the computer through a cable or a network, or a disk array formed by connecting a plurality of hard disk drives. A control program such as an operating system, various application programs, and display data of various screens associated with these programs are stored in the storage device 25.

The memory 26 is a work memory required when the CPU 27 executes processing. The CPU 27 performs overall control of each unit of the computer by loading a program stored in the storage device 25 to the memory 26 and executing the processing according to the program.

The communication unit 28 is a network interface to perform transmission control of various kinds of information through the network 13. The display 29 displays various screens corresponding to the operation of the input device 30, such as a mouse or a keyboard. The screen has an operation function based on the graphical user interface (GUI). Each computer that forms the client terminal 11 or the like receives an input of an operation instruction from the input device 30 through the screen.

In the following explanation, for the sake of distinction, a suffix "A" is attached to the reference numeral of each unit of the computer that forms the client terminal 11, and a suffix "B" is attached to the reference numeral of each unit of the computer that forms the information analysis assistance server 12.

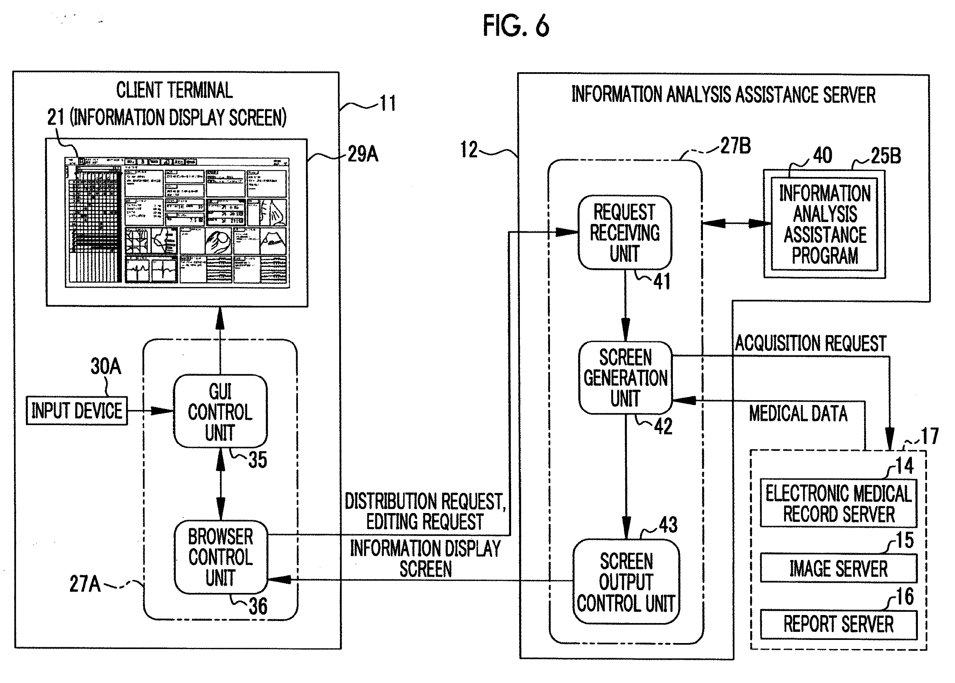

In Fig. 6, when a web browser is started, the CPU 27A of the client terminal 11 cooperates with the memory 26 or the like to function as a GUI control unit 35 and a browser control unit 36.

The GUI control unit 35 displays various screens on the display 29A, and receives an operation instruction that is input from the input device 30A through various screens. As the operation instruction, there are an instruction to distribute the information display screen 21 to the information analysis assistance server 12 and an instruction to edit the information display screen 21. The GUI control unit 35 outputs the received operation instruction to the browser control unit 36.

The browser control unit 36 controls the operation of the web browser. The browser control unit 36 issues a distribution request according to the distribution instruction from the GUI control unit 35 and an editing request according to the editing instruction to the information analysis assistance server 12.

The browser control unit 36 receives image data of the information display screen 21 from the information analysis assistance server 12. The browser control unit 36 reproduces the information display screen 21 to be displayed on the web browser based on the image data, and outputs the information display screen 21 to the GUI control unit 35. The GUI control unit 35 displays the information display screen 21 on the display 29A.

An information analysis assistance program 40 is stored in the storage device 25B of the information analysis assistance server 12. The information analysis assistance program 40 is an application program for operating a computer, which forms the information analysis assistance server 12, as an information analysis assistance device, and corresponds to the operation program of the information analysis assistance device.

When the information analysis assistance program 40 is started, the CPU 27B of the information analysis assistance server 12 cooperates with the memory 26 or the like to function as a request receiving unit 41, a screen generation unit 42, and a screen output control unit 43.

The request receiving unit 41 receives various requests from the client terminal 11. The request receiving unit 41 outputs a distribution request and an editing request to the screen generation unit 42.

The screen generation unit 42 issues an acquisition request to the server group 17 in response to the distribution request, and acquires medical data transmitted from the server group 17 in response to the acquisition request. The screen generation unit 42 generates the information display screen 21 based on the acquired medical data. In addition, the screen generation unit 42 edits the information display screen 21 in response to the editing request. The screen generation unit 42 outputs the generated or edited information display screen 21 to the screen output control unit 43. The screen output control unit 43 outputs the information display screen 21 to the client terminal 11 that is a source of the distribution request or the editing request.

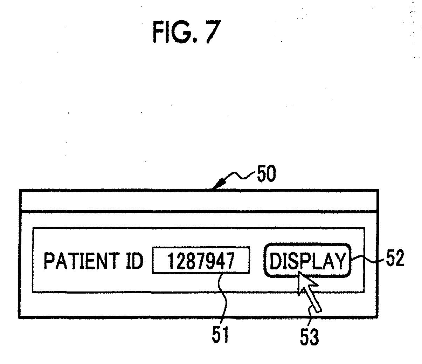

In Fig. 7, a patient ID input screen 50 is a screen that is displayed first on the web browser of the display 29A after a doctor performs authentication by accessing the information analysis assistance server 12 through the client terminal 11. An input box 51 for inputting a patient ID and a display button 52 are provided on the patient ID input screen 50. Instead of the patient ID, the name of the target patient may be input.

If a patient ID of a patient to be subject to medical treatment (hereinafter, referred to as a target patient) is input to the input box 51 and the display button 52 is selected by a cursor 53, a distribution request including the patient ID of the target patient is transmitted to the information analysis assistance server 12 from the client terminal 11.

In the acquisition request issued to the server group 17 by the screen generation unit 42, the patient ID of the target patient is assigned as a search key. The server group 17 transmits the electronic medical record 18 including the patient ID of the target patient, the examination image 19, and the medical report 20 to the screen generation unit 42 as medical data. The screen generation unit 42 generates the information display screen 21 shown in Fig. 8 based on the medical data of the target patient from the server group 17.

In Fig. 8, the information display screen 21 has three display regions of a first display region 60, a second display region 61, and a third display region 62. The first display region 60 is disposed on the left side of the information display screen 21, and occupies, for example, about 1/3 of the area of the information display screen 21. A table 63 is displayed in the first display region 60. The second display region 61 is disposed side by side on the right side of the first display region 60, and occupies, for example, about 2/3 of the area of the information display screen 21. In the second display region 61, medical data of the target patient is displayed as information.

The third display region 62 is disposed on the upper side of the information display screen 21. Patient ID, name, sex, age, date of birth, and taste of the target patient are collectively displayed on the left side of the third display region 62. In addition, the medical condition of the target patient that should be kept in mind in particular at the time of diagnosis, such as "high blood pressure" or "diabetes", is displayed. A department to which a doctor who has logged in to the information analysis assistance server 12 belongs and the name of the doctor are displayed on the right side of the third display region 62.

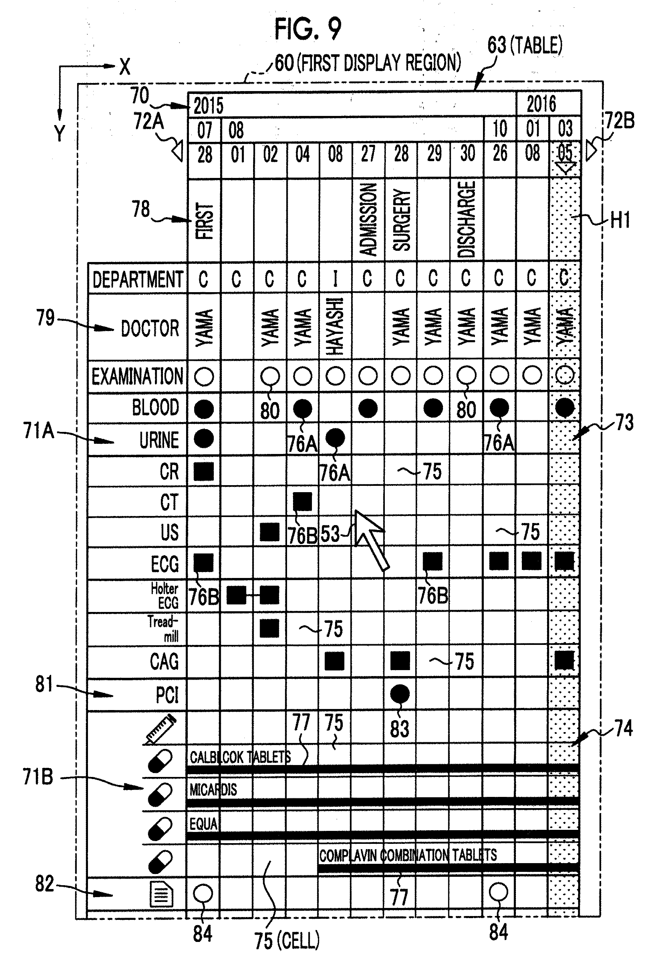

In Fig. 9, on the X axis in the row direction of the table 63, a date on which medical data has been acquired is assigned as one of two types of attributes to classify the medical data. On the Y axis in the column direction of the table 63 perpendicular to the X axis, items of medical data are assigned as the other one of the two types of attributes. Specifically, the table 63 has a date field 70, in which a date on which medical data has been acquired is displayed, and a first item field 71A and a second item field 71B, in which items of the medical data are displayed. The date field 70 is provided at the top of the table 63 along the X-axis direction. The item fields 71A and 71B are provided on the left side of the table 63 along the Y-axis direction.

The date field 70 is divided into three stages in the Y-axis direction. Year, month, and day are displayed in the upper stage, middle stage, and lower stage, respectively. A date can be selected by the cursor 53. If a date is selected by the cursor 53, an editing request of the information display screen 21 is transmitted to the information analysis assistance server 12 from the client terminal 11.

Figs. 8 and 9 show a state in which "2016. 03. 05", which is the latest date on which the medical data of the target patient has been acquired, is selected as shown by hatching H1. In response to a distribution request, the screen generation unit 42 first generates, for example, the information display screen 21 shown in Figs. 8 and 9 where the latest date, on which the medical data of the target patient has been acquired, is disposed at the right end of the date field 70 and the latest date has been selected.

In the date field 70, past dates are disposed leftward. A date back button 72A and a date feed button 72B are disposed on the left and right sides of the date field 70. If the date back button 72A is selected by the cursor 53, dates of the date field 70 are returned by one day toward the past. On the other hand, if the date feed button 72B is selected by the cursor 53, dates of the date field 70 are advanced by one day toward the latest date.

A medical examination is assigned to the first item field 71A as an item, and a drug is assigned to the second item field 71B as an item. In the item fields 71A and 71 B, characters or illustrations that express the items directly are displayed. Specifically, the initial letter of each subject examination, such as "blood" and "urine", is displayed in the item of a subject examination, such as a blood test or a urine test, of the first item field 71 A. An alphabet abbreviation of each image examination, such as "CT" or "CAG", is displayed in the item of an image examination, such as a CT examination or a CAG examination. In the item of a drug of the second item field 71 B, illustrations of a syringe and capsule tablets are displayed.

A plurality of items in the item fields 71A and 71B can be selected by the cursor 53. If an item is selected by the cursor 53, an editing request of the information display screen 21 is transmitted to the information analysis assistance server 12 from the client terminal 11.

An examination history field 73 showing whether or not a medical examination has been performed is provided on the right side of the first item field 71 A. A dosing history field 74 showing whether or not a drug has been administered is provided on the right side of the second item field 71 B. The history fields 73 and 74 are a group of a plurality of rectangular cells 75 that are intersections of the dates of the date field 70 and the items of the item fields 71 A and 71 B.

A mark 76 indicating that a medical examination has been performed is displayed on the cell 75 of the examination history field 73. The mark 76 is displayed only in the cell 75 corresponding to a medical examination performed on the day. Nothing is displayed in the cell 75 corresponding to a medical examination that has not been performed. In this case, the cell 75 corresponding to a medical examination that has not been performed is blanked. The screen generation unit 42 assigns the mark 76 to the cell 75 of the examination history field 73 based on the date of each subject examination of the electronic medical record 18 and the date of each image examination of the examination image 19.

There are two types of marks 76, and a circular mark 76A is used for the subject examination and a square mark 76B is used for the image examination. Through the mark 76, it can be seen at a glance on which day what kind of medical examination has been performed. For example, in the case of "2015. 07. 28", the mark 76A is displayed for "blood" and "urine", and the mark 76B is displayed for "CR" and "ECG". Therefore, it can be seen that a blood test, urinalysis, a CR examination, and an ECG examination were performed in "2015. 07. 28".

In the cell 75 of the dosing history field 74, a bar 77 indicating that a drug has been administered is displayed together with the name of the drug. The bar 77 is displayed only in the cell 75 corresponding to the drug administered on the day. Nothing is displayed in the cell 75 corresponding to a drug that has not been administered. In this case, the cell 75 corresponding to a drug that has not been administered is blanked. The screen generation unit 42 assigns the bar 77 to the cell 75 of the dosing history field 74 based on the day of administration of the drug in the electronic medical record 18.

Through the bar 77, it can be seen at a glance on which day what kind of drug has been administered. In the example shown in Fig. 9, it can be seen that all drugs excluding "COMPLAVIN COMBINATION TABLETS" have been administered in the entire period of the dates displayed in the date field 70 and the administration of "COMPLAVIN COMBINATION TABLETS" has started from "2015. 08. 08".

Similar to the items of the item fields 71A and 71B, a plurality of cells 75 of the examination history field 73 in which the mark 76 is displayed can also be selected by the cursor 53. On the other hand, the cell 75 of the dosing history field 74 cannot be selected. If the cell 75 of the examination history field 73 in which the mark 76 is displayed is selected by the cursor 53, an editing request of the information display screen 21 is transmitted to the information analysis assistance server 12 from the client terminal 11. In addition, the date field 70 is also referred to as a table head or a box head, each of the item fields 71 A and 71 B is also referred to as a table side or a stub, and each of the history fields 73 and 74 is also referred to as a table body or a field.

Between the date field 70 and the first item field 71A and the examination history field 73, an event history field 78 and a medical history column 79 are disposed. In the event history field 78, characters briefly indicating various events, such as a first visit, admission and discharge, and surgery that have occurred on the day, specifically, the initial letter of each event is displayed. In Fig. 9, "first" indicating a first visit is displayed in "2015. 07. 28", "admission" indicating that the patient was admitted is displayed in "2015. 08. 27", and "discharge" indicating that the patient was discharged is displayed in "2015. 08. 30".

The medical history column 79 is divided into three stages in the Y-axis direction. An initial of the medical department of a doctor in charge of the medical examination, such as "C (cardiovascular department)" and "I (internal medicine)", is displayed in the upper stage, an initial of the name of a doctor in charge of the medical examination, such as "Yama (Yamamoto Ichiro)", is displayed in the middle stage, and a circular mark 80 indicating that a medical examination has been performed is displayed in the lower stage. For example, since "C", "Yama", and the mark 80 are displayed in "2015. 07. 28", it can be seen that Yamamoto Ichiro of the cardiovascular department has performed the medical examination in "2015. 07. 28". On the other hand, since only "C" is displayed in "2015. 08. 01", it can be seen that only the Holter ECG examination was performed without a medical examination in "2015.08.01".

Between the first item field 71A and the examination history field 73 and the second item field 71B and the dosing history field 74, a percutaneous coronary intervention (PCI) history column 81 is disposed. In addition, a note history column 82 is disposed below the second item field 71B and the dosing history field 74. A circular mark 83 indicating that a doctor has written a comment about the PCI is displayed in the PCI history column 81. In addition, a circular mark 84 indicating that a doctor has written a note is displayed in the note history column 82. The PCI history column 81 is displayed only for a target patient for whom the PCI has been performed. The note history column 82 is also displayed only in a case where a doctor has written a note.

The cells 75 of the PCI history column 81 and the note history column 82 cannot be selected. All of the mark 76A of the examination history field 73, the mark 80 of the medical history column 79, the mark 83 of the PCI history column 81, and the mark 84 of the note history column 82 are circular. However, these are displayed so as to be distinguished from each other by changing the presence of filling or the display color.

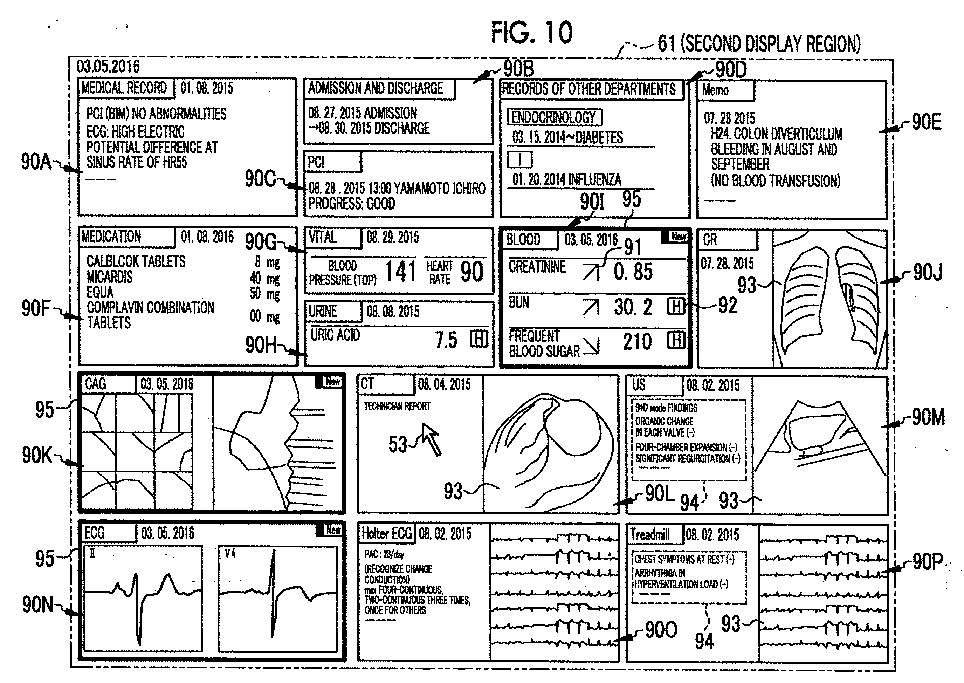

In a case where the date of "2016. 03. 05" is selected by the cursor 53 as shown by hatching H1 in Figs. 8 and 9, a plurality of windows 90A to 90P are displayed side by side in the second display region 61 as shown in Fig. 10. Here, the date is an attribute assigned to the X axis in the row direction. Accordingly, the case where a date is selected by the cursor 53 corresponds to a first selection method, and the display format of the second display region 61 in Fig. 10 corresponds to a first display format.

In Fig. 10, an item name of medical data, an acquisition date of the medical data, and an outline of the medical data are displayed in the windows 90A to 90P. The windows 90A to 90P are roughly divided into windows 90G to 90P to display the examination results of medical examinations, a window 90F (item name: "medication") to display drugs and dosages, and the other windows 90A to 90E of medical data. The windows 90G to 90P to display the examination results of medical examinations are divided into a window 90G to display the examination results of vital signs, windows 90H and 90I to display the examination results of subject examinations, and windows 90J to 90P to display the examination results of image examinations.

Examination items, such as "heart rate", "uric acid", and "creatinine", and examination values thereof, an arrow 91 indicating whether the examination value has increased or decreased compared with the previous examination value, and a mark 92 indicating that the examination value is higher than the reference value are displayed in the windows 90G, 90H, and 90I.

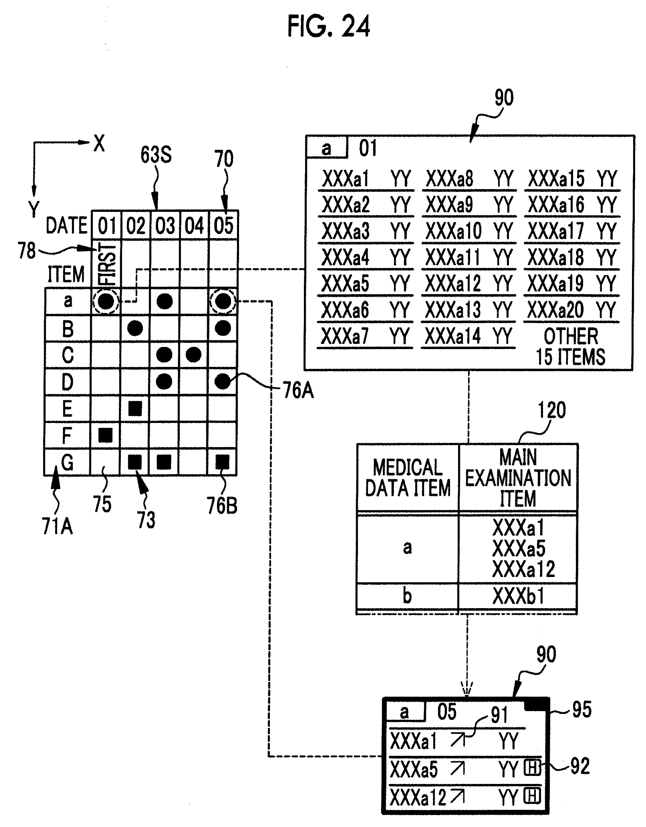

Only main examination items set in advance of all examination items are displayed in the windows 90G, 90H, and 90I. In the storage device 25B, a main examination item table 120 (refer to Fig. 24) in which main examination items are registered for each item of an examination is stored. The screen generation unit 42 selects examination items to be displayed in the windows 90G, 90H, and 90I based on the main examination item table 120.

On the other hand, a reduced image 93 of the examination image 19 and findings 94 of the radiologist described in the medical report 20 are displayed in the windows 90J to 90P to display the examination results of image examinations.

In Fig. 10, the window 90H (item name: "urine") to display the examination results of urinalysis and the window 90I (item name: "blood") to display the examination results of the blood test are displayed as windows to display the examination results of subject examinations. In addition, the window 90J (item name: "CR") to display the examination results of the CR examination, the window 90K (item name: "CAG") to display the examination results of the CAG examination, the window 90L (item name: "CT") to display the examination results of the CT examination, the window 90M (item name: "US") to display the examination results of the US examination, the window 90N (item name: "ECG") to display the examination results of the ECG examination, the window 900 (item name: "Holter ECG") to display the examination results of the Holter ECG examination, and the window 90P (item name: "Treadmill") to display the examination results of the load ECG examination are displayed as windows to display the examination results of image examinations.

Drugs and dosages, that is, a part of prescription, is displayed in the window 90F. Medical record description (item name: "medical record"), events (item name: "admission and discharge"), comments on the PCI (item name: "PCI"), history (item name: "other department record"), and notes (item name: "Memo") are displayed in the other windows 90A to 90E of medical data, respectively.

The layout, such as the order or size, of each window 90 may be set in advance by the information analysis assistance program 40, or may be freely customized by the doctor.

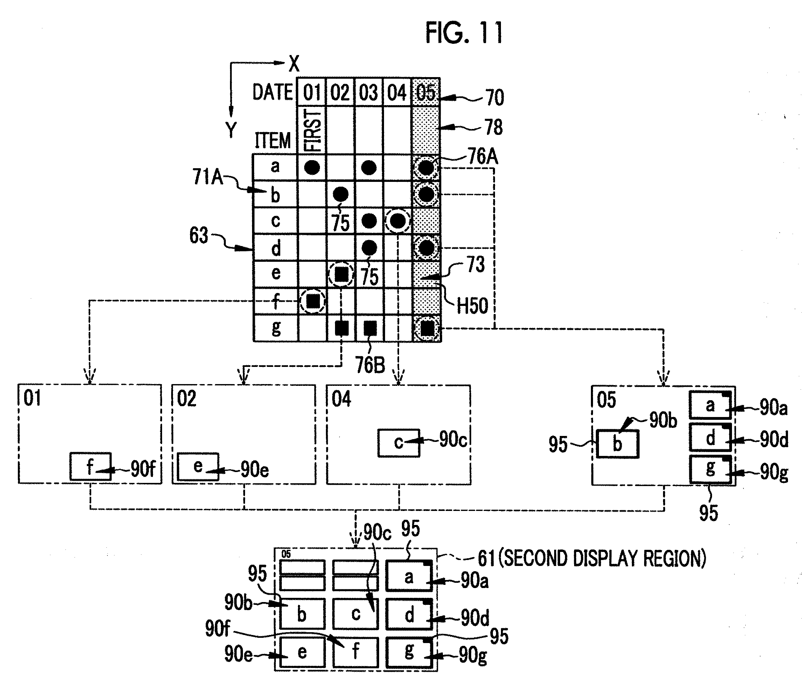

Fig. 11 is an explanatory diagram showing how the examination results are displayed in the second display region 61 in a case where the date is selected. In Fig. 11, for convenience of explanation, the table 63 shown in the upper stage is obtained by simplifying the table 63 shown in Figs. 8 and 9 by removing the second item field 71B, the dosing history field 74, the medical history column 79, the PCI history column 81, the note history column 82, and the like. Dates of five days of "01" to "05" are displayed in the date field 70, and items of seven medical examinations of "a" to "g" are displayed in the first item field 71A. In addition, the mark 76 is displayed in the cell 75 of the examination history field 73, in the same manner as in table 63 shown in Figs. 8 and 9. For example, in "05", the marks 76A and 76B are displayed in the items "a", "b", "d", and "g". Accordingly, it can be seen that medical examinations of "a", "b", "d", and "g" were performed in "05".

Fig. 11 shows a case where "05", which is the latest date, has been selected by the first selection method, as shown by hatching H50 in the table 63. In the lower stage, the second display region 61 in a case where "05" is selected is shown. Windows 90a to 90g displayed in the second display region 61 are windows to display the examination results of the medical examinations "a" to "g", respectively.

The latest examination results on the selected date are displayed in the windows 90a to 90g of the second display region 61, respectively. More specifically, for a medical examination for which there is an examination result acquired on the selected date, the examination result acquired on the selected date is displayed in each of the windows 90a to 90g. On the other hand, for a medical examination for which there is no examination result acquired on the selected date, the latest examination results among the examination results acquired before the selected date are displayed in the windows 90a to 90g.

In the case shown in Fig. 11, medical examinations for which there are examination results acquired on the selected date "05" are "a", "b", "d", and "g", as can be seen from the table 63. For these medical examinations, as shown on the right side in the middle stage, examination results acquired on the date "05" are displayed in the windows 90a, 90b, 90d, and 90g where the examination results of the medical examinations are displayed.

On the other hand, medical examinations for which there is no examination result acquired on the selected date "05" are "c", "e", and "f", as can be seen from the table 63. For these medical examinations, as shown on the left side from the center of the middle stage, the latest examination results among the examination results acquired before the date "05" are displayed in the windows 90c, 90e, and 90f where the examination results of the medical examinations are displayed. That is, an examination result acquired on the date "04" is displayed in the window 90c where the examination result of the medical examination "c" is displayed, an examination result acquired on the date "02" is displayed in the window 90e where the examination result of the medical examination "e" is displayed, and an examination result acquired on the date "01" is displayed in the window 90f where the examination result of the medical examination "f" is displayed. In this manner, as shown in the lower stage, the windows 90a, 90b, 90d, and 90g to display the examination results acquired on the date "05" and the windows 90c, 90e, and 90f to display the examination results acquired on dates other than the date "05" are displayed side by side in the second display region 61.

For example, in a case where "03" is selected by the cursor 53, examination results acquired on the date "03" are displayed in the windows 90a, 90c, 90d, and 90g since there are examination results acquired on the date "03" for the medical examinations "a", "c", "d", and "g". However, since there is no examination result acquired on the date "03" for the medical examinations "b", "e", and "f", examination results acquired on the date "02" are displayed in the windows 90b and 90e and examination results acquired on the date "01" are displayed in the window 90f.

In this case, the window 90 where the examination result acquired on the date selected by the cursor 53 is displayed (in the example shown in Fig. 11, the window 90a or the like) and the window 90 (the example of Fig. 11 window 90c etc.) where other examination results acquired on dates other than the date selected by the cursor 53 are mixed in the second display region 61. For this reason, it is not distinguishable in this state which window 90 is the window 90 where the examination result acquired on the date selected by the cursor 53 is displayed.

Therefore, in the second display region 61, a new frame 95 indicating that the examination result was acquired on the date selected by the cursor 53 is displayed in the window 90 where the examination result acquired on the date selected by the cursor 53 is displayed (in Fig. 11, the windows 90a, 90b, 90d, and 90g).

In the example shown in Fig. 10, examination results acquired in "2016. 03. 05" selected by the cursor 53 are a blood test, a CAG examination, and an ECG examination. Therefore, examination results of the blood test, the CAG examination, and the ECG examination acquired in "2016. 03. 05" are displayed in the windows 90I, 90K, and 90N to display the examination results, respectively. In addition, the new frame 95 is displayed in each of the windows 90I, 90K, and 90N. In the windows 90G to 90P to display the examination results of medical examinations excluding the windows 90I, 90K, and 90N described above, the latest examination results among the examination results acquired before "2016. 03. 05" are displayed. For example, the examination result of the CT examination acquired on "2015. 08. 04" is displayed in the window 90L to display the examination result of the CT examination. The new frame 95 is not displayed in such a window 90.

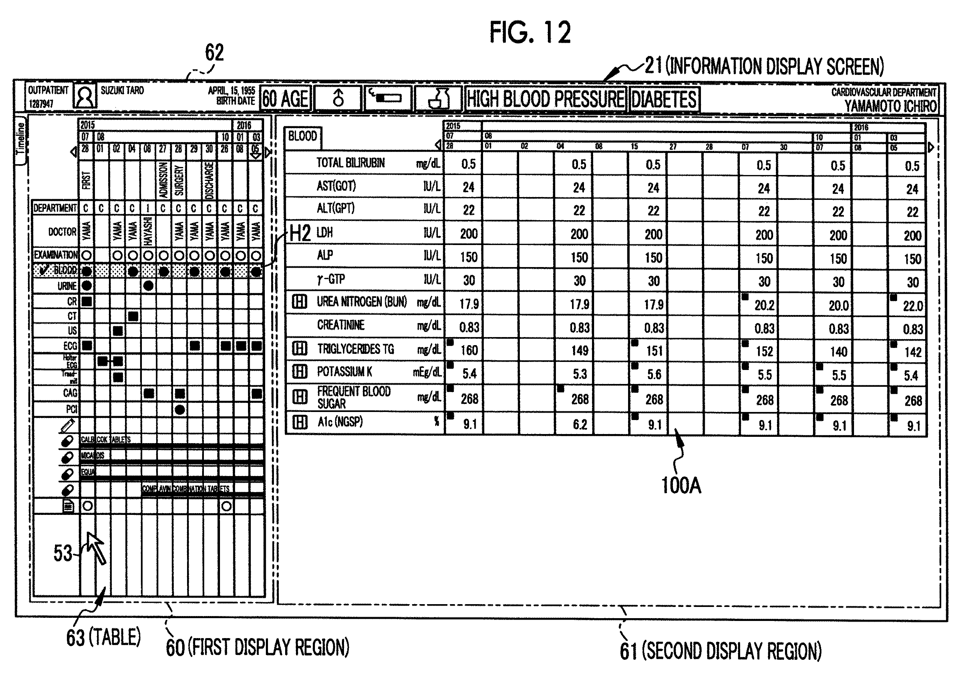

Fig. 12 shows the information display screen 21 in a case where the item of blood test in the first item field 71A is selected by the cursor 53, as shown by hatching H2. In this case, a time-series change table 100A showing a time-series change in the examination value of each examination item of the blood test is displayed in the second display region 61. Here, the item of blood test is an attribute assigned to the Y axis in the column direction. Accordingly, the case where the item of blood test is selected by the cursor 53 corresponds to a second selection method, and the display format of the second display region 61 in Fig. 12 corresponds to a second display format.

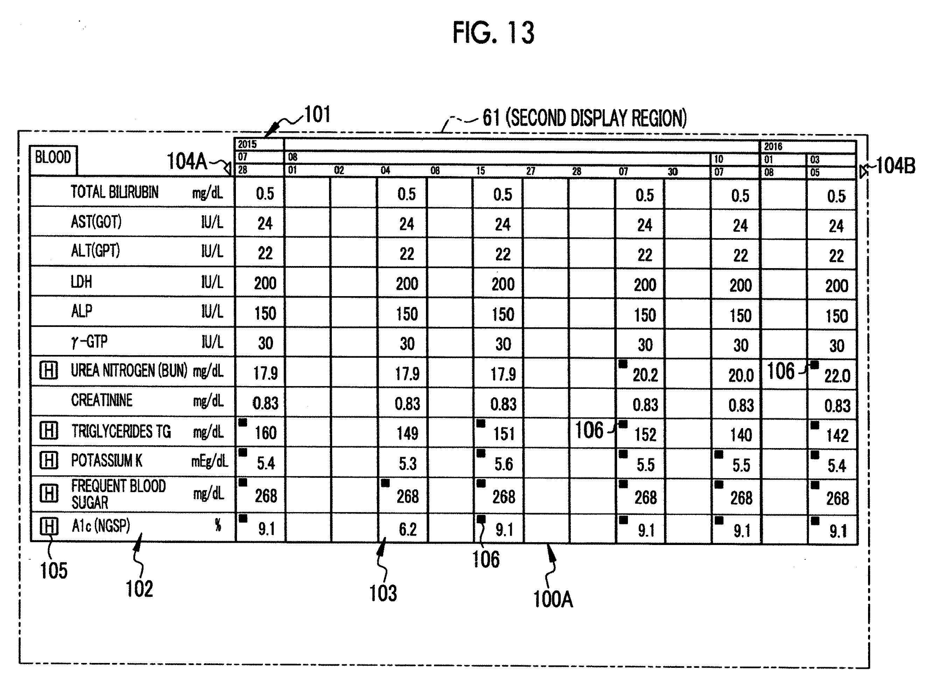

In Fig. 13 obtained by enlarging the second display region 61 in Fig. 12, the time-series change table 100A has a date field 101 similar to the date field 70 in the table 63, an examination item field 102 in which examination items and their units are displayed, and an examination value history field 103 which is provided on the right side of the examination item field 102 and in which examination values are written.

A plurality of selected examination items including main examination items, among all of the examination items, are displayed in the examination item field 102. A date back button 104A and a date feed button 104B, which are the same as in the table 63, are disposed on the left and right sides of the date field 101. For an examination item having an examination value higher than the reference value, the same mark 105 as the mark 92 in Fig. 10 is displayed in the examination item field 102, and a square mark 106 is displayed in the examination value history field 103.

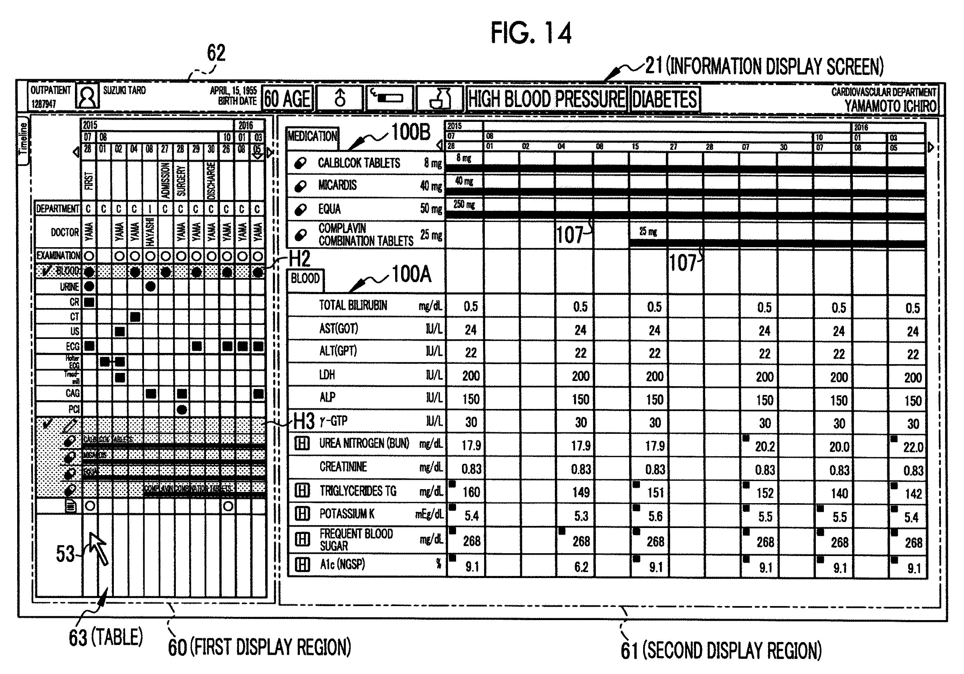

Fig. 14 shows the information display screen 21 in a case where the item of blood test in the first item field 71 A shown in Fig. 12 and the item of drugs in the second item field 71B are selected by the cursor 53, as shown by hatching H2 and hatching H3. In this case, in addition to the time-series change table 100A showing a time-series change in the examination value of each examination item of the blood test, a time-series change table 100B showing time-series changes in drugs and dosages is displayed in the second display region 61. Here, the item of drugs is also an attribute assigned to the Y axis in the column direction, similar to the item of blood test. Accordingly, the case where the items of blood test and drugs are selected by the cursor 53 corresponds to a case where a plurality of items are selected in the second selection method, and the display format of the second display region 61 in Fig. 14 corresponds to the second display format in a case where a plurality of items are selected.

The time-series change table 100B is obtained by enlarging the display of the second item field 71B and the dosing history field 74. Drugs and dosages and the same bar 107 as the bar 77 in the dosing history field 74 are displayed. In this case, the date field 101, the date back button 104A, and the date feed button 104B are common in the time-series change tables 100A and 100B. Although not shown, in a case where the item of image examination is selected by the cursor 53, the reduced image 93 of the examination image 19 is displayed side by side in time series in the second display region 61.

As a method of displaying time-series changes in medical data, instead of or in addition to the time-series change table 100 described above, for example, a line graph obtained by plotting the examination values of each day and connecting these with a line on a two-dimensional plane having a date on the horizontal axis and an examination value on the vertical axis may be displayed.

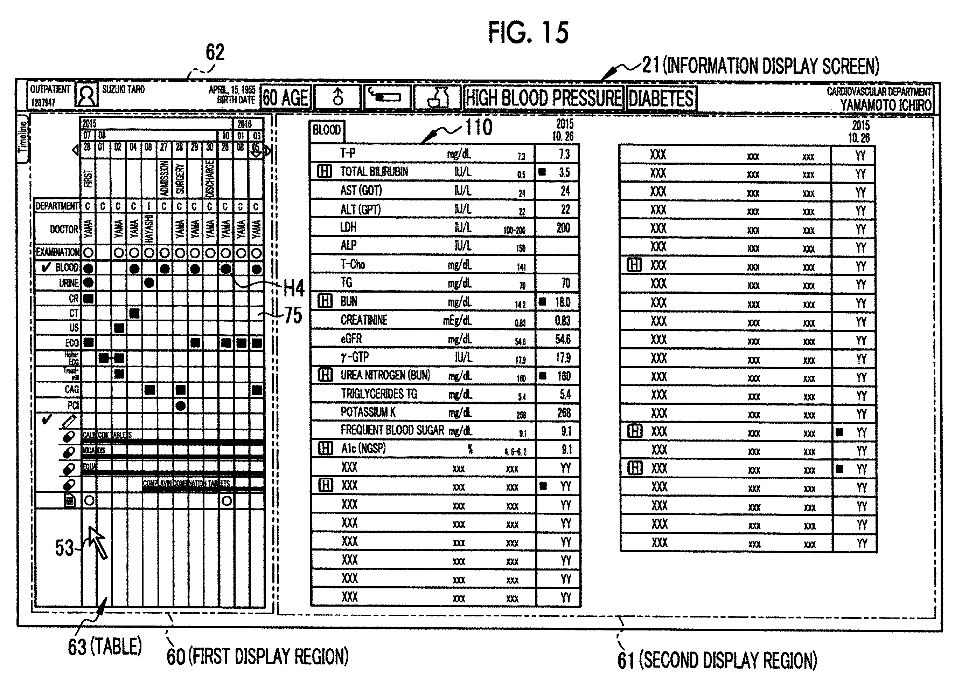

Fig. 15 shows the information display screen 21 in a case where the cell 75 of the item of blood test in "2015. 10. 26" is selected by the cursor 53, as shown by hatching H4. In this case, a details table 110 showing the examination values of all examination items of the blood test in "2015. 10. 26" is displayed in the second display region 61. Here, the cell 75 is an intersection between a date and an item that are two kinds of attributes. Accordingly, the case where the cell 75 is selected by the cursor 53 corresponds to a third selection method, and the display format of the second display region 61 in Fig. 15 corresponds to a third display format.

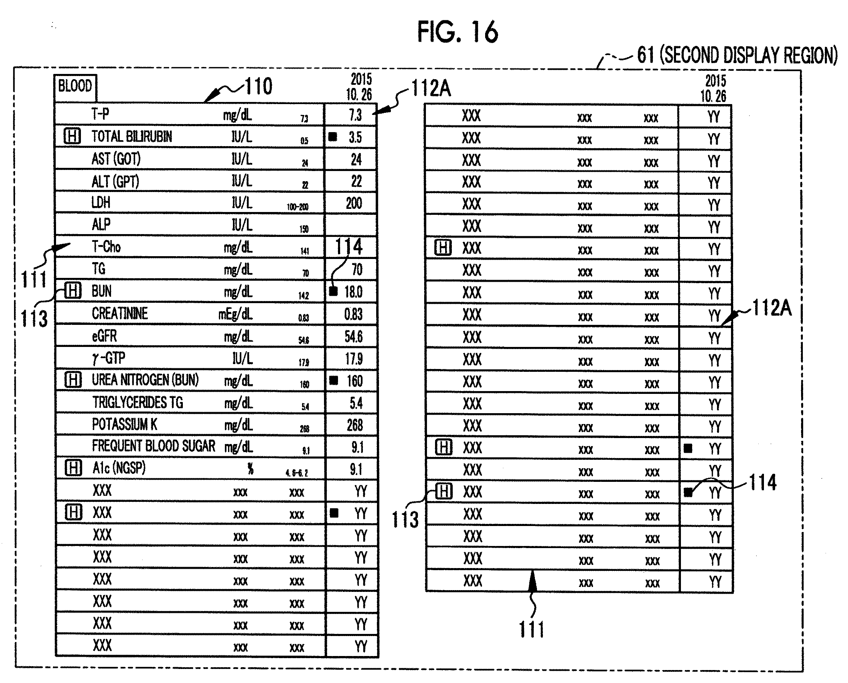

In Fig. 16 obtained by enlarging the second display region 61 in Fig. 15, the details table 110 is divided into two left and right parts. That is, the details table 110 has an examination item field 111, in which examination items, their units, and reference values of examination values are displayed, and an examination value field 112A, in which examination values are written. All examination items are displayed in the examination item field 111. In addition, a date corresponding to the selected cell 75 is displayed above the examination value field 112A. For an examination item having an examination value higher than the reference value, the same mark 113 as the mark 92 in Fig. 10 or the mark 105 in Fig. 13 is displayed in the examination item field 102, and the same mark 114 as the mark 106 in Fig. 13 is displayed in the examination value field 112A.

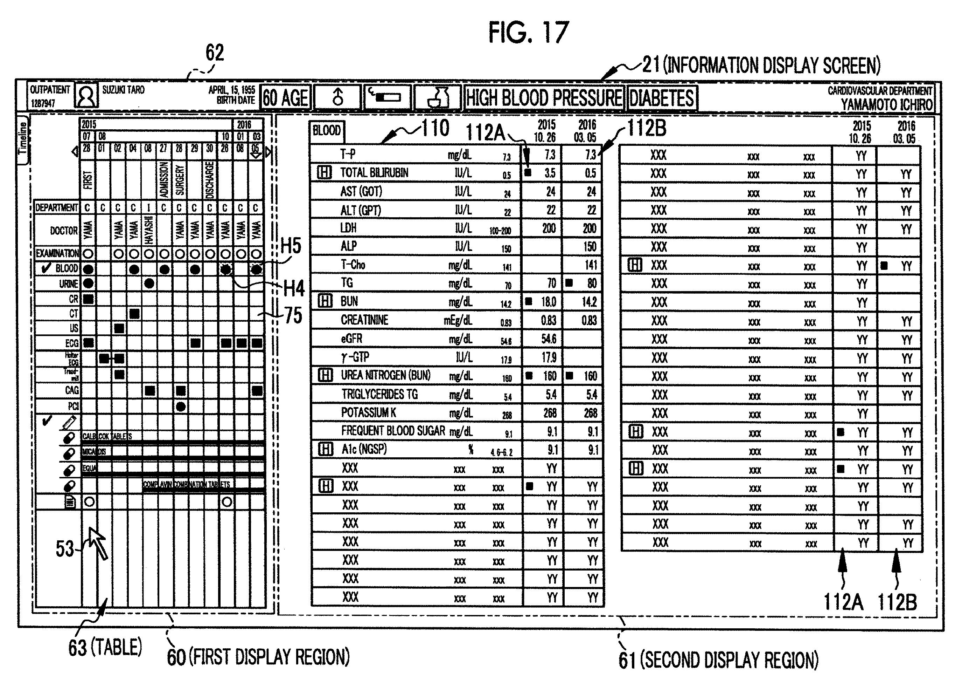

Fig. 17 shows the information display screen 21 in a case where the cell 75 of the item of blood test in "2015. 10. 26" and the cell 75 of the item of blood test in "2016. 03. 05" are selected by the cursor 53, as shown by hatching H4 and hatching H5. In this case, in the details table 110 of the second display region 61, an examination value field 112B in which the examination value of the blood test in "2016. 03. 05" is written is added beside the examination value field 112A in which the examination value of the blood test in "2015. 10. 26" is written. Here, the case where the cell 75 of the item of blood test in "2015. 10. 26" and the cell 75 of the item of blood test in "2016. 03. 05" are selected by the cursor 53 corresponds to a case where a plurality of cells 75 are selected in the third selection method, and the display format of the second display region 61 in Fig. 17 corresponds to the third display format in a case where a plurality of cells 75 are selected.

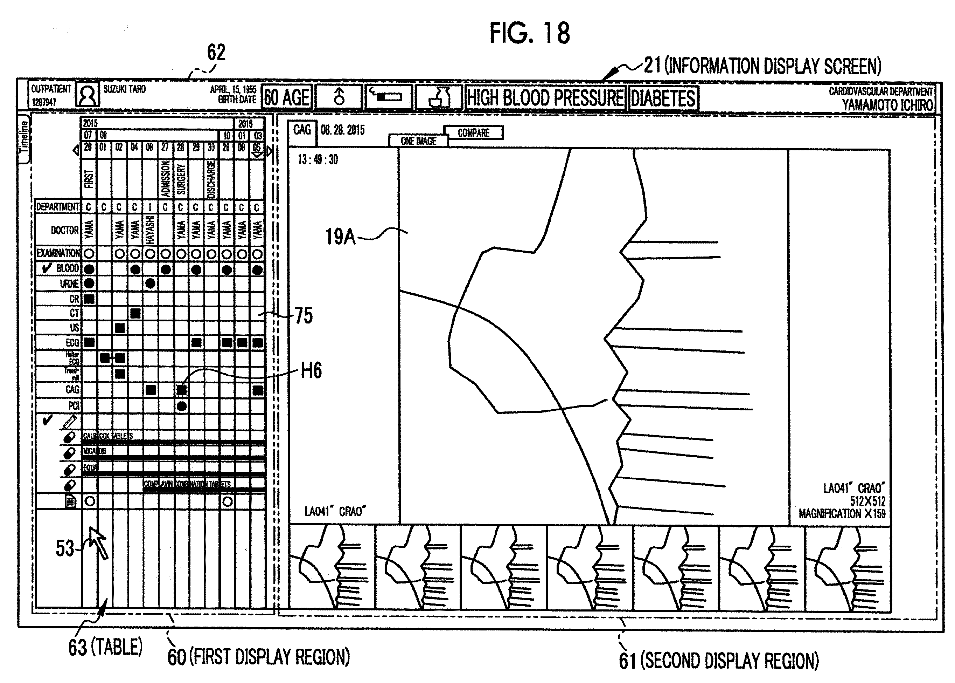

Fig. 18 shows the information display screen 21 in a case where the cell 75 of the item of CAG examination in "2015. 08. 28" is selected by the cursor 53, as shown by hatching H6. In this case, an examination image 19A obtained by the CAG examination in "2015. 08. 28" is displayed in the second display region 61. As in the case shown in Fig. 15, this case also corresponds to the third selection method, and the display format of the second display region 61 corresponds to the third display format.

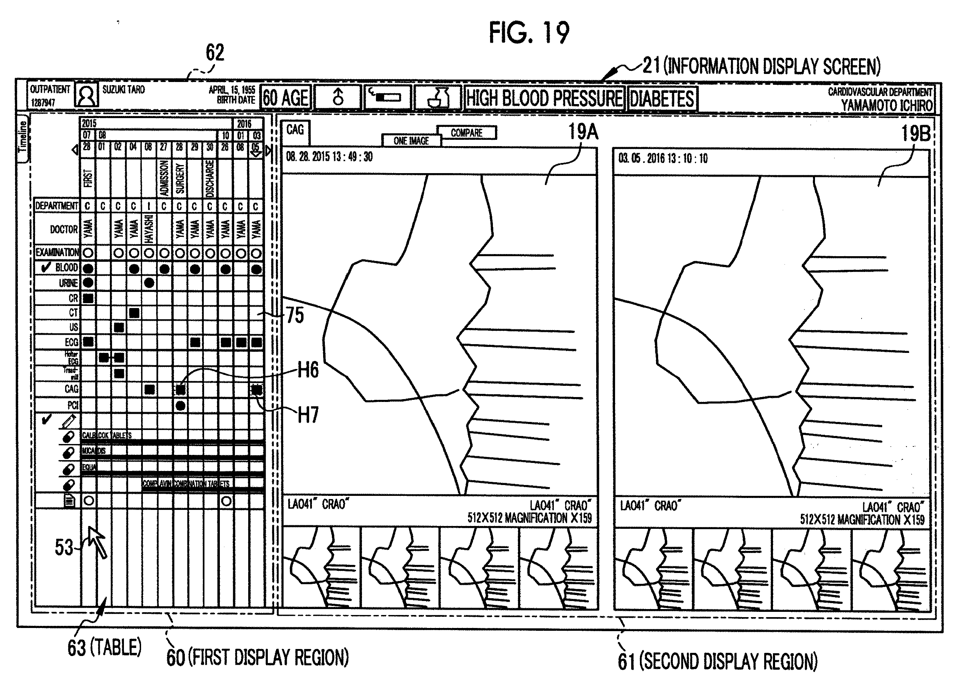

Fig. 19 shows the information display screen 21 in a case where the cell 75 of the item of CAG examination in "2015. 08. 28" and the cell 75 of the item of CAG examination in "2016. 03. 05" are selected by the cursor 53, as shown by hatching H6 and hatching H7. In this case, in addition to the examination image 19A obtained by the CAG examination in "2015. 08. 28", an examination image 19B obtained by the CAG examination in "2016. 03.05" is displayed in the second display region 61. As in the case shown in Fig. 17, this case also corresponds to a case where a plurality of cells 75 are selected in the third selection method, and the display format of the second display region 61 corresponds to the third display format in a case where a plurality of cells 75 are selected.

As described above with reference to Figs. 8 to 10 and 12 to 19, in a case where a date in the table 63 is selected (first selection method; Figs. 8 to 10), a case where an item is selected (second selection method; Figs. 12 to 14), and a case where a plurality of cells 75 are selected (third selection method; Figs. 15 to 19), the medical data is displayed in three different display formats in the second display region 61.

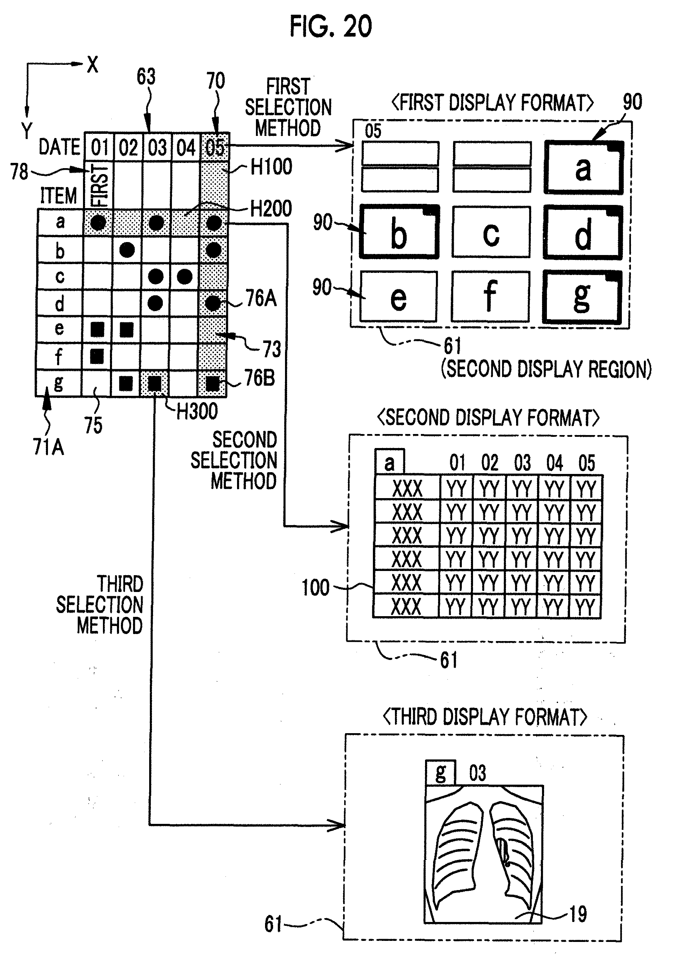

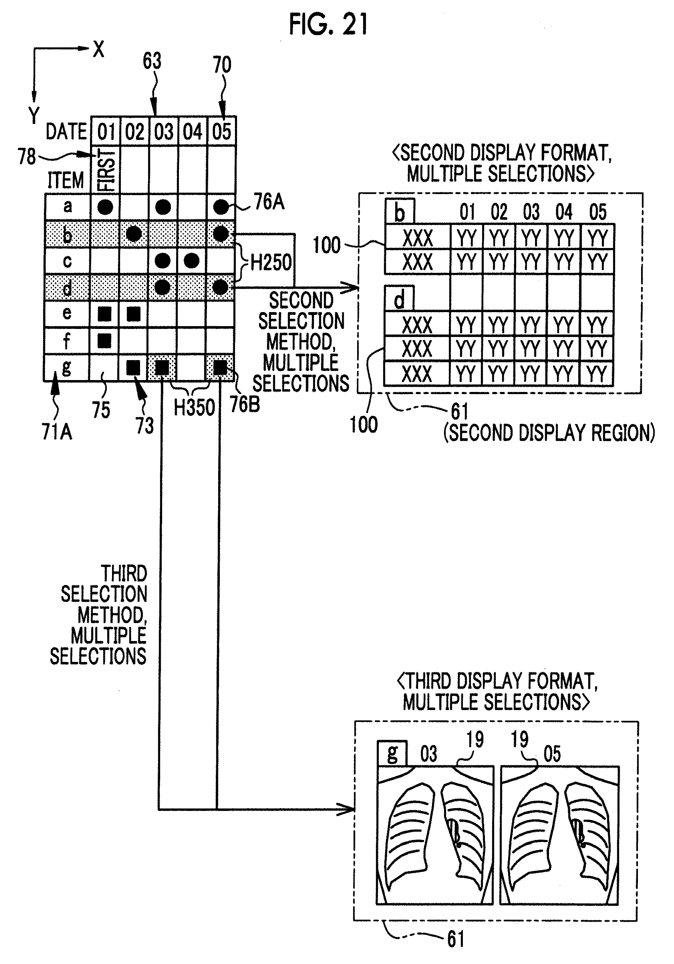

In Figs. 20 and 21, the display format of the information display screen 21 corresponding to each selection method is summarized using the simple table 63 shown in Fig. 11.

In Fig. 20, as shown by hatching H100, in a case where a date is selected by the first selection method, the screen generation unit 42 sets the first display format to display the windows 90 showing the outline of each piece of medical data side by side in the second display region 61, as shown in the upper stage.

As shown by hatching H200, in a case where an item is selected by the second selection method, the screen generation unit 42 sets the second display format to display the time-series change table 100 showing time-series changes in medical data in the second display region 61, as shown in the middle stage.

As shown by hatching H300, in a case where the cell 75 is selected by the third selection method, the screen generation unit 42 sets the third display format to display the details (here, the examination image 19) of medical data in the second display region 61, as shown in the lower stage.

In Fig. 21, as shown by hatching H250, in a case where a plurality of items are selected by the second selection method, the screen generation unit 42 displays the time-series change tables 100 of the plurality of selected pieces of medical data side by side in the second display region 61, as shown in the upper stage. In addition, as shown by hatching H350, in a case where a plurality of cells 75 are selected by the third selection method, the screen generation unit 42 displays the details (here, the examination image 19) of medical data corresponding to the plurality of selected cells 75 side by side in the second display region 61, as shown in the lower stage.

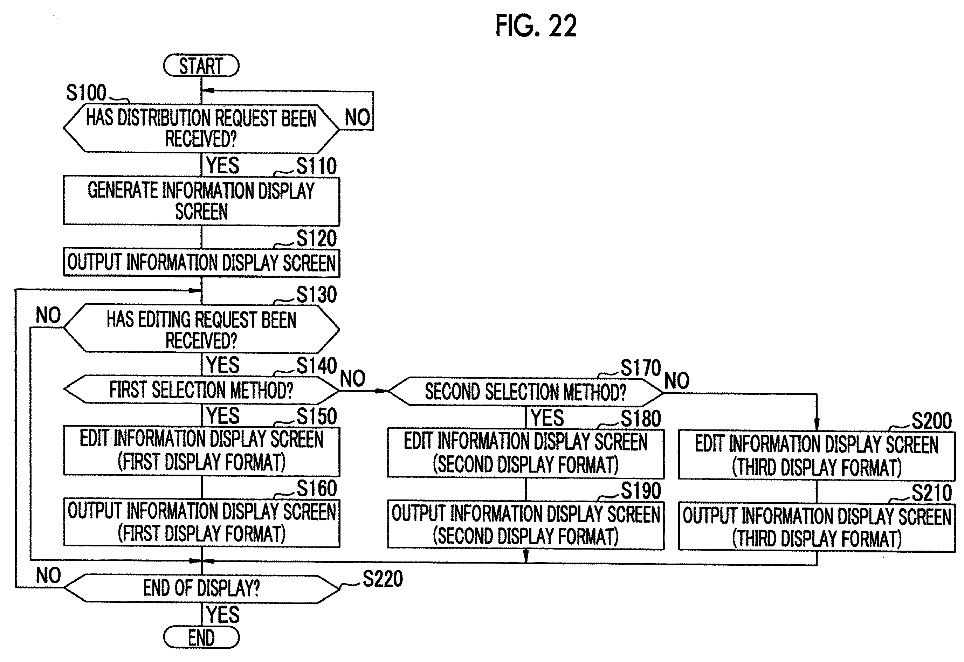

Hereinafter, the operation of the above configuration will be described with reference to the flowchart shown in Fig. 22. First, a doctor performs authentication by accessing the information analysis assistance server 12 through the client terminal 11. After the authentication, the patient ID input screen 50 shown in Fig. 7 is displayed on the display 29A of the client terminal 11.

The doctor inputs a patient ID of a target patient in the input box 51 of the patient ID input screen 50, and selects the display button 52 with the cursor 53. Then, a distribution request of the information display screen 21 is transmitted to the information analysis assistance server 12 from the client terminal 11.

In the information analysis assistance server 12, the distribution request is received by the request receiving unit 41 (YES in step S100). The distribution request is output to the screen generation unit 42. Then, an acquisition request is issued to the server group 17 from the screen generation unit 42, and the medical data of the target patient transmitted from the server group 17 in response to the acquisition request is acquired by the screen generation unit 42.

As shown in step S110, the information display screen 21 based on the medical data of the target patient is generated by the screen generation unit 42. The generated information display screen 21 is output to the client terminal 11, which is a source of the distribution request, by the screen output control unit 43 (step S120). In this case, the information display screen 21 is in a state of the first display format in which a plurality of windows 90 showing the outline of the medical data of the target patient are displayed in the second display region 61 based on the first selection method, in which the latest date on which the medical data of the target patient has been acquired is selected, as shown in Figs. 8 to 10.

In the client terminal 11, the information display screen 21 from the information analysis assistance server 12 is received by the browser control unit 36, and the information display screen 21 is displayed on the display 29A by the GUI control unit 35.

The doctor views the information display screen 21, and selects a date, an item, or the cell 75 in the table 63 with the cursor 53 when necessary. Then, an editing request of the information display screen 21 is transmitted to the information analysis assistance server 12 from the client terminal 11.

In the information analysis assistance server 12, the editing request is received by the request receiving unit 41 (YES in step S130). The editing request is output to the screen generation unit 42. In a case where a date in the table 63 is selected by the cursor 53, that is, in the case of the first selection method (YES in step S140), the screen generation unit 42 edits the information display screen 21 in the first display format in which the windows 90 showing the outline of medical data are displayed side by side in the second display region 61 (step S150). The information display screen 21 edited in the first display format is output to the client terminal 11, which is a source of the editing request, by the screen output control unit 43 (steps 160).

In the first display format, the medical condition of the target patient can be comprehensively understood through the window 90 showing the outline of medical data. In the first display format, in a case where there is no examination result acquired on the date selected by the cursor 53, the latest examination result among the examination results acquired before the selected date is displayed. Accordingly, it is possible to compare the examination result acquired on the selected date with the examination result acquired before the selected date. In this case, since the new frame 95 is displayed in the window 90 where the examination result acquired on the date selected by the cursor 53 is displayed, the examination result acquired on the date selected by the cursor 53 and the other examination results can be distinguished.

In addition, in the first display format, the windows 90A, 90B, and 90D showing the medical record description, events, and history are displayed in the second display region 61. Therefore, it is possible to understand the medical condition of the target patient more clearly.

In a case where an item in the table 63 is selected by the cursor 53, that is, in the case of the second selection method (NO in step S140 and YES in step S170), the screen generation unit 42 edits the information display screen 21 in the second display format in which the time-series change table 100 showing time-series changes in medical data is displayed in the second display region 61 (step S180). In a case where a plurality of items are selected, the time-series change tables 100 of the plurality of selected pieces of medical data are displayed side by side in the second display region 61. The information display screen 21 edited in the second display format is output to the client terminal 11, which is a source of the editing request, by the screen output control unit 43 (step S190).

In the second display format, changes in the medical condition of the target patient can be accurately understood through the time-series change table 100. In addition, in the case where a plurality of items are selected, the time-series change tables 100 of the plurality of selected pieces of medical data are displayed side by side in the second display region 61. Therefore, it is possible to check the cause-and-effect relationship between a plurality of pieces of medical data, for example, by selecting the items of blood test and drugs and determining the therapeutic effect after drug administration.

In a case where the cell 75 in the table 63 is selected by the cursor 53, that is, in the case of the third selection method (NO in steps S140 and S170), the screen generation unit 42 edits the information display screen 21 in the third display format in which the details of medical data is displayed in the second display region 61 (step S200). In a case where a plurality of cells 75 are selected, the details of medical data corresponding to the plurality of selected cells 75 are displayed side by side in the second display region 61. The information display screen 21 edited in the third display format is output to the client terminal 11, which is a source of the editing request, by the screen output control unit 43 (step S210). The process of steps S130 to S210 is continued until the end of the display of the information display screen 21 (YES in step S220).

In the third display format, the medical condition of the target patient can be understood in detail. In addition, in the case where a plurality of cells 75 are selected, the details of medical data corresponding to the plurality of selected cells 75 are displayed side by side in the second display region 61. Therefore, it is possible to compare a plurality of pieces of medical data in detail. For example, it is possible to determine whether or not the medical condition of the target patient has improved by comparing the examination image 19 in the previous CAG examination with the examination image 19 in the current CAG examination.

Since the medical data is displayed in the second display region 61 in three different display formats according to each selection method, it is possible to understand information from many angles. For example, from the first visit to admission, more extensive examination results are viewed as the first display format using the first selection method. Immediately before and after surgery, more detailed examination results are viewed as the third display format using the third selection method. In the follow-up as an outpatient after surgery, time-series changes in the examination results are observed as the second display format using the second selection method. Therefore, it is possible to analyze the examination results by freely switching to the point of view adapted to the medical phase.

In addition, when making a diagnosis, an entire image is taken in more extensive examination results as the first display format using the first selection method, an item of interest is checked in more detailed examination results as the third display format using the third selection method, and time-series changes in the examination results are checked in order to determine the medication as the second display format using the second selection method. That is, even in the same medical phase, it is possible to analyze the examination results by freely switching to the point of view according to the purpose.

The display format switching operation is just selecting a date, an item, or the cell 75 using the cursor 53. In addition, since the first display region 60 and the second display region 61 are disposed side by side on the same screen instead of separate screens, an operation of display switching between the first display region 60 and the second display region 61 is not necessary. Therefore, it is possible to advance the analysis of examination results efficiently.

In a case where a doctor makes a diagnosis by analyzing the examination results of medical examinations, the point of view of the analysis of the examination results changes with the medical phase. Therefore, there is a high need to understand information from many angles. Therefore, it is possible to meet the needs to understand information from many angles in the medical field by offering the information analysis assistance with the medical data as information as in the present embodiment.

In addition, since a doctor is very busy, the doctor may want to understand information without effort if possible. In the invention, since the display format switching operation is simple and there is no need of a complicated screen switching operation, it is possible to meet the needs to understand information without effort. Just by performing a clicking operation on the table 63 as an index of examination results, three kinds of display formats of the second display region 61 can be selected without a complicated operation. Therefore, the operation is easy. The ease of operation is an effect achieved since the table 63 as an index of examination results is displayed in the first display region 60.

In the first embodiment described above, the new frame 95 is displayed in the window 90 where examination results acquired on the date selected by the cursor 53 are displayed, so that the window 90 where examination results acquired on the date selected by the cursor 53 are displayed is distinguished from the other windows 90. However, instead of or in addition to the new frame 95, the window 90 where examination results acquired on the date selected by the cursor 53 are displayed may be distinguished from the other windows 90, for example, by displaying the outer frame of the window 90 where examination results acquired on the date selected by the cursor 53 so as to blank or by setting the background color of the window 90 where examination results acquired on the date selected by the cursor 53 to a different color from the other windows 90.

In the first embodiment described above, in the first display format, examination results acquired on the selected date and the latest examination results, among examination results acquired before the selected date, are displayed in the second display region 61. However, only the examination results acquired on the selected date may be displayed in the second display region 61. In this case, it is not necessary to display the new frame 95.

In Figs. 17, 19, and 21, cases where a plurality of cells 75 of the same item, such as a blood test and a CAG examination, are selected are illustrated. However, a plurality of cells 75 of different items may be selected.

### [Second embodiment]

In the first embodiment described above, in a case where the cell 75 is selected by the third selection method, the details of medical data are displayed in the second display region 61. However, in addition to the case where the cell 75 is selected, a case where the window 90 is selected in the first display format may be a trigger of detailed display of medical data.

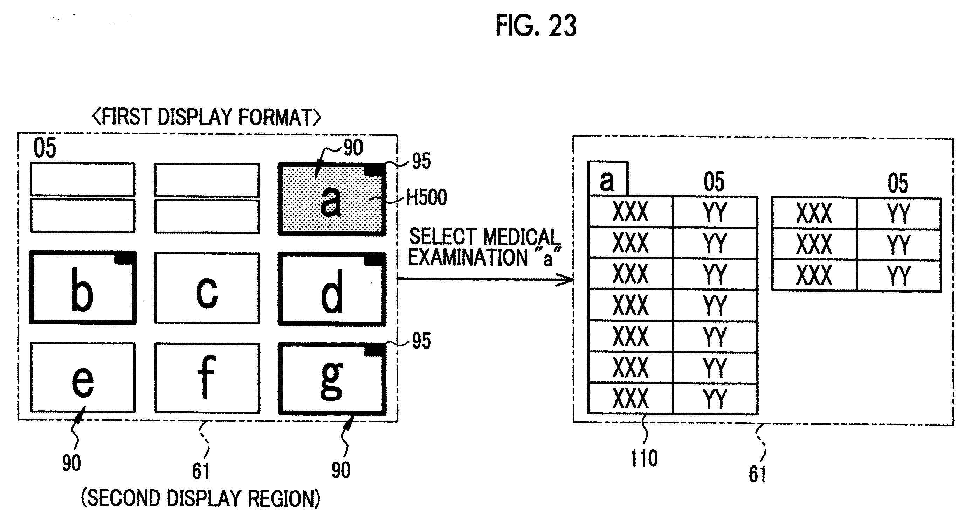

Specifically, as shown on the left side of Fig. 23, in the first display format, in a case where the window 90 to display the examination result of a medical examination "a" is selected by the cursor 53 as shown by hatching H500, the screen generation unit 42 switches the display of the second display region 61 to the display of the details of the examination result of the medical examination "a" (here, the details table 110 of the examination result of the medical examination "a") as in the third display format. Thus, it is possible to improve the convenience of the doctor by preparing two kinds of triggers of the detailed display of medical data.

### [Third embodiment]

In the first embodiment described above, in the first display format, only the main examination items set in advance among all examination items are displayed in the windows 90G, 90H, and 90I to display examination results including a plurality of examination items. However, examination items to be displayed may be switched according to whether or not the corresponding examination result is an examination result acquired during the first visit.

Specifically, as shown in the upper stage in Fig. 24, in a case where the examination result of the medical examination "a" of "0 1", which is the date of the first visit, is displayed in the window 90, the screen generation unit 42 performs display so as to include examination items other than the main examination items (in this case, XXXa1, XXXa5, and XXXa12) set in the main examination item table 120. On the other hand, in a case where an examination result acquired at a time other than the first visit, for example, the examination result of the medical examination "a" of "05", which is a date other than the first visit, is displayed in the window 90, the screen generation unit 42 displays only the main examination items as shown in the lower stage.

At the first visit, in order to make a more accurate definitive diagnosis, a large amount of determination materials are required. Therefore, in the case of displaying the examination results acquired during the first visit in the second display region 61 as in the present embodiment, items other than the main examination items are also displayed, so that it is possible to provide a doctor with a sufficient amount of determination materials for making a more accurate definitive diagnosis.

In a case where the day has passed from the first visit, the number of medical examinations that have been performed is generally larger than that at the first visit. Accordingly, the display space of each window 90 becomes narrow. For this reason, the amount of information to be displayed in the window 90 is limited. However, by displaying only the main examination items in the case of displaying the examination results acquired at a time other than the first visit in the second display region 61, it is possible to provide a doctor with the minimum necessary information in the limited display space.

### [Fourth embodiment]

In the first display format, according to the number of windows 90 (examination results) to be displayed side by side in the second display region 61, the number of examination items of one window 90 (examination result) to be displayed in the second display region 61 may be increased or decreased.

Specifically, in a case where the number of windows 90 to be displayed in the second display region 61 is relatively small, the screen generation unit 42 increases the number of examination items to be displayed in one window 90. On the other hand, in a case where the number of windows 90 to be displayed in the second display region 61 is relatively large, the screen generation unit 42 decreases the number of examination items of examination results to be displayed in one window 90.

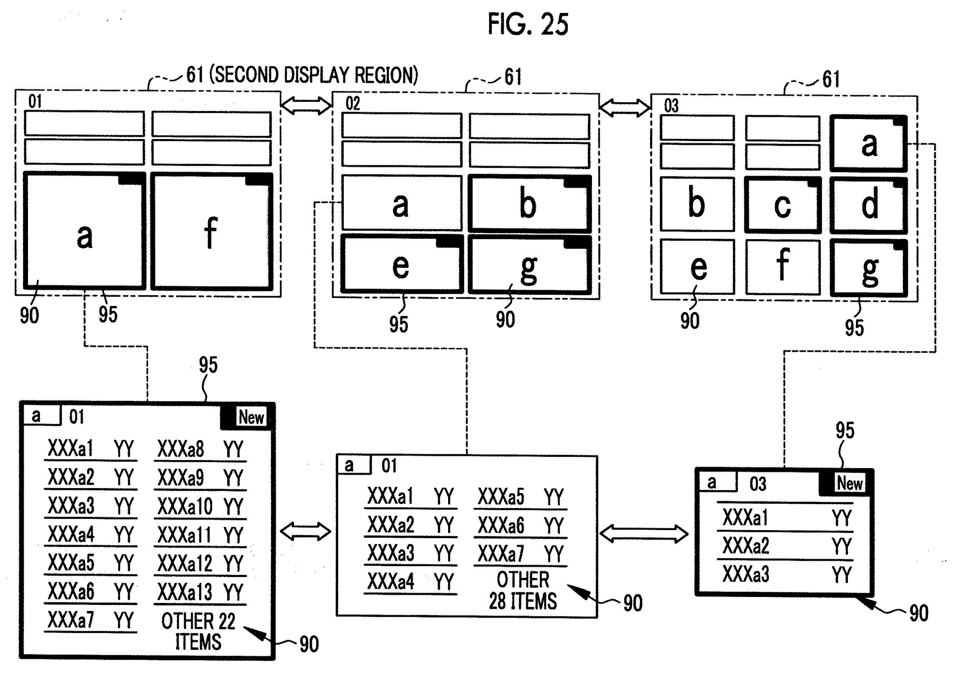

More specifically, as shown on the left side of Fig. 25, in a case where two medical examinations "a" and "f" are displayed in the windows 90 to be displayed in the second display region 61, a total of thirteen examination items of XXXa1 to XXXa13 are displayed in the window 90 where the examination result of the medical examination "a" is displayed.

As shown in the middle of Fig. 25, in a case where four medical examinations "a", "b", "e", and "g" are displayed in the windows 90 to be displayed in the second display region 61, a total of seven examination items of XXXa1 to XXXa7 are displayed in the window 90 where the examination result of the medical examination "a" is displayed. That is, the number of examination items to be displayed in the window 90 where the examination result of the medical examination "a" is displayed is reduced by six from that in the case where the number of windows 90 is two as shown on the left side of Fig. 25.

In addition, as shown on the right side of Fig. 25, in a case where seven medical examinations "a" to "g" are displayed in the windows 90 to be displayed in the second display region 61, a total of three examination items of XXXa1 to XXXa3 are displayed in the window 90 where the examination result of the medical examination "a" is displayed. That is, the number of examination items to be displayed in the window 90 where the examination result of the medical examination "a" is displayed is reduced by four from that in the case where the number of windows 90 is four as shown in the middle of Fig. 25.

Thus, in the first display format, the number of examination items of one examination result to be displayed in the second display region 61 is increased or decreased according to the number of examination results to be displayed side by side in the second display region 61, so that it is possible to appropriately display examination results according to the display space. In addition, in the case of reducing the number of examination items to be displayed in the window 90 to the minimum number, only the main examination items may be displayed as in the third embodiment described above.

### [Fifth embodiment]

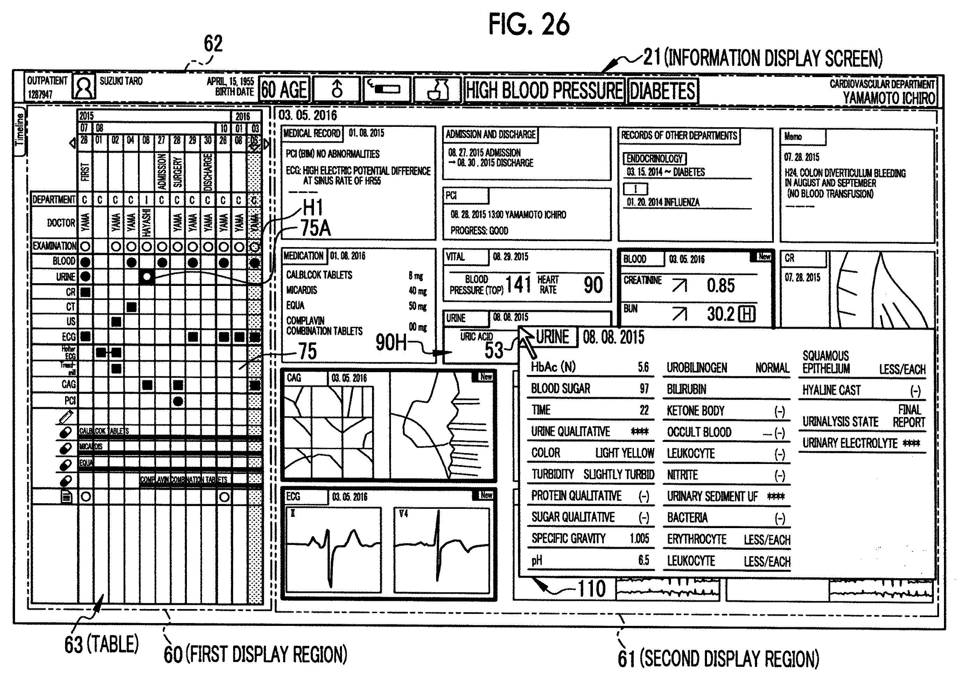

In this embodiment, as shown in Fig. 26, for example, in a case where the cursor 53 is superimposed on the window 90H to display the examination result of urinalysis, the screen generation unit 42 may popup-display the details table 110 of the examination result of urinalysis in the second display region 61. In addition, the screen generation unit 42 displays a cell 75A corresponding to the examination result of urinalysis where the details table 110 is popup-displayed (in this case, the cell 75A corresponding to the urinalysis of "2015. 08. 08") so as to be distinguished from other cells 75, for example, by solarizing the mark 76A and portions other than the mark 76.

Thus, in the first display format, by popup-displaying the details of medical data in the second display region 61, the details of certain medical data and the outline of other medical data can be simultaneously displayed in the second display region 61. Accordingly, these can be compared. In addition, since the cell 75A corresponding to medical data whose details are popup-displayed is displayed so as to be distinguished from other cells, it can be seen at a glance which medical data is being popup-displayed in detail.

In addition, the superposition of the cursor 53 on the window 90 is a trigger to popup-display the details of medical data. Instead of this, the details of medical data may be popup-displayed in a case where the window 90 is selected by the cursor 53 or in a case where the cursor 53 is superimposed on the window 90 and is right-clicked. In addition, the cell 75A corresponding to medical data whose details are popup-displayed may be made to blink so as to be distinguished from the other cells 75.

In each of the embodiments described above, a date is assigned to the X axis in the row direction of the table 63, and an item is assigned to the Y axis in the column direction of the table 63. However, conversely, an item may be assigned to the X axis in the row direction of the table 63, and a date may be assigned to the Y axis in the column direction of the table 63.

The hardware configuration of a computer, which forms the information analysis assistance server 12 corresponding to the information analysis assistance device of the invention, can be modified in various ways. For example, in order to improve the processing capacity or reliability, the information analysis assistance server 12 may be formed by a plurality of server computers that are separated from each other as hardware. For example, the functions of the request receiving unit 41, the screen generation unit 42, and the screen output control unit 43 may be distributed in two server computers. In this case, the two server computers form the information analysis assistance device.

In the first embodiment described above, the case has been exemplified in which the information analysis assistance server 12 generates the information display screen 21 and the information display screen 21 is reproduced on the client terminal 11 side based on the image data of the information display screen 21 from the information analysis assistance server 12 and is displayed on the display 29A. However, medical data that is a source of the generation of the information display screen 21 may be transmitted to the client terminal 11 from the information analysis assistance server 12, and the information display screen 21 may be generated on the client terminal 11 side. In this case, the screen generation unit 42 is constructed in the CPU 27A of the client terminal 11.

Each functional unit constructed in the CPU 27B of the information analysis assistance server 12 excluding the screen output control unit 43 may be constructed in the CPU 27A of the client terminal 11, so that the client terminal 11 operates as an information analysis assistance device. In this case, the request receiving unit 41 receives a distribution instruction or the like from the GUI control unit 35 instead of a distribution request or the like. In addition, the screen generation unit 42 outputs the generated information display screen 21 to the GUI control unit 35. In this case, the GUI control unit 35 has a function of the screen output control unit 43.

Thus, the hardware configuration of a computer can be appropriately changed according to the required performance, such as processing capacity, safety, or reliability. Needless to say, in order to ensure the safety or reliability, an application program, such as the information analysis assistance program 40, may be duplicated or may be stored in a plurality of storage devices in a distributed manner, without being limited to hardware.

Although the embodiment in which the information analysis assistance server 12 is used in one medical facility has been described in each of the above embodiments, the information analysis assistance server 12 may be made to be able to be used in a plurality of available medical facilities.

In each of the embodiments described above, the information analysis assistance server 12 is communicably connected to the client terminal 11, which is installed in one medical facility, through the network 13, such as a LAN, and provides the information display screen 21 in response to a distribution request from the client terminal 11 or the like. In order to make the information analysis assistance server 12 available in a plurality of medical facilities, the information analysis assistance server 12 is communicably connected to each client terminal 11 installed in the plurality of medical facilities, for example, through a wide area network (WAN), such as the Internet or a public communication network. Then, the information analysis assistance server 12 receives distribution requests from the client terminals 11 in the plurality of medical facilities through the WAN, and provides the information display screen 21 to the client terminals 11. In the case of using a WAN, it is preferable to construct a virtual private network (VPN) or to use a communication protocol with a high security level, such as hypertext transfer protocol secure (HTTPS), in consideration of information security.

In this case, the electronic medical record 18, the examination image 19, and the medical report 20 are managed for each medical facility. In this case, the installation location and management entity of the information analysis assistance server 12 may be a data center managed by a company that is different from the medical facilities, or may be one of the plurality of medical facilities, for example.

Although the medical data has been exemplified as information in each of the embodiments described above, the invention is not limited thereto. Various kinds of data, for example, process management data of product testing before shipment or print calibration data of publications may be handled as information.

In the case of process management data of product testing before shipment, the date of a process and the kind of test before shipment are assigned to the row-direction axis and the column-direction axis in the table, respectively, as attributes. For example, outlines of the results of tests before shipment are displayed side by side in the first display format, time-series changes in measured values obtained in tests before shipment are displayed in the second display format, and the details of the results of tests before shipment are displayed in the third display format. In the case of print calibration data of publications, the date of calibration and the kind of calibration are assigned to the row-direction axis and the column-direction axis in the table, respectively, as attributes. For example, outlines of the results of calibrations are displayed side by side in the first display format, time-series changes in calibration results are displayed in the second display format, and the details of calibration results are displayed in the third display format.

In the invention, it is also possible to appropriately combine the above-described various embodiments or various modification examples. Without being limited to the embodiments described above, it is needless to say that various configurations can be adopted without departing from the scope of the invention. In addition to the program, the invention also extends to a storage medium that stores the program.

## Claims

1. An information analysis assistance device, comprising:
a screen generation unit that generates an information display screen having a first display region to display a table, in which two kinds of attributes for classifying information are assigned to an axis in a row direction or an axis in a column direction, and a second display region to display the information; and
a screen output control unit that controls an output of the information display screen,
wherein the screen generation unit displays the information in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attributes is selected.

2. The information analysis assistance device according to claim 1,
wherein the information is medical data of a plurality of items acquired in a medical procedure of one patient.

3. The information analysis assistance device according to claim 2,
wherein the two kinds of attributes are a date on which the medical data has been acquired and an item of the medical data,
the screen generation unit assigns the date to one of the axis in the row direction and the axis in the column direction and assigns the item to the other one,
in a case where the date is selected by one of the first and second methods, the screen generation unit sets the display format to a first display format in which the medical data items acquired on a selected date are displayed side by side in the second display region,
in a case where the item is selected by the other one of the first and second methods, the screen generation unit sets the display format to a second display format in which time-series changes in the medical data are displayed in the second display region, and
in a case where the cell is selected by the third method, the screen generation unit sets the display format to a third display format in which details of the medical data are displayed in the second display region.

4. The information analysis assistance device according to claim 3,
wherein, in the first display format, in a case where the medical data of the second display region is selected, the screen generation unit displays details of the selected medical data in the second display region in the same manner as in the third display format.

5. The information analysis assistance device according to claim 3 or 4,
wherein, in the first selection method or the second selection method, a plurality of the items are selectable, and
in a case where a plurality of the items are selected, the screen generation unit displays time-series changes in the plurality of pieces of selected medical data side by side in the second display region in the second display format.

6. The information analysis assistance device according to any one of claims 3 to 5,
wherein, in the third selection method, a plurality of the cells are selectable, and
in a case where a plurality of the cells are selected, the screen generation unit displays details of the medical data corresponding to the plurality of selected cells side by side in the second display region in the third display format.

7. The information analysis assistance device according to any one of claims 3 to 6,
wherein, in the first display format, in a case where there is no medical data acquired on the date selected by the first selection method or the second selection method, the screen generation unit displays the latest medical data of the medical data acquired before the selected date in the second display region.

8. The information analysis assistance device according to claim 7,
wherein the screen generation unit displays the medical data acquired on the date selected by the first selection method or the second selection method so as to be distinguished from the other pieces of medical data.

9. The information analysis assistance device according to any one of claims 3 to 8,
wherein the screen generation unit assigns at least a medical examination performed on the patient and a drug administered to the patient, as the items, to one of the axis in the row direction and the axis in the column direction, and
the screen generation unit displays an examination result of the medical examination, the drug, and a dose in the second display region as the medical data.

10. The information analysis assistance device according to claim 9,
wherein the examination result has a plurality of examination items.

11. The information analysis assistance device according to claim 9,
wherein, in the first display format, in a case where the examination result acquired during a first visit is displayed in the second display region, the screen generation unit performs display so as to include examination items other than main examination items set in advance of the plurality of examination items, and
in a case where the examination result acquired at a time other than the first visit is displayed in the second display region, the screen generation unit displays only the main examination items.

12. The information analysis assistance device according to claim 9 or 10,
wherein, in the first display format, the screen generation unit increases or decreases the number of examination items of one examination result to be displayed in the second display region according to the number of examination results to be displayed side by side in the second display region.

13. The information analysis assistance device according to any one of claims 3 to 12,
wherein, in the first display format, the screen generation unit popup-displays details of the medical data in the second display region.

14. The information analysis assistance device according to claim 13,
wherein the screen generation unit displays the cell corresponding to the medical data of which details are popup-displayed so as to be distinguished from the other cells.

15. The information analysis assistance device according to any one of claims 2 to 14,
wherein, in the first display format, the screen generation unit further displays a medical record description, an event including admission and discharge, and history in the second display region as the medical data.

16. An operation method of an information analysis assistance device, comprising:
a screen generation step of generating an information display screen having a first display region to display a table, in which two kinds of attributes for classifying information are assigned to an axis in a row direction or an axis in a column direction, and a second display region to display the information; and
a screen output control step of controlling an output of the information display screen,
wherein, in the screen generation step, the information is displayed in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attributes is selected.

17. An operation program of an information analysis assistance device causing a computer to execute:
a screen generation function of generating an information display screen having a first display region to display a table, in which two kinds of attributes for classifying information are assigned to an axis in a row direction or an axis in a column direction, and a second display region to display the information; and
a screen output control function of controlling an output of the information display screen,
wherein, by the screen generation function, the information is displayed in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attributes is selected.

18. An information analysis assistance system comprising an information analysis assistance device and a client terminal connected to the information analysis assistance device through a network, comprising:
a screen generation unit that generates an information display screen having a first display region to display a table, in which two kinds of attributes for classifying information are assigned to an axis in a row direction or an axis in a column direction, and a second display region to display the information; and
a screen output control unit that controls an output of the information display screen,
wherein the screen generation unit displays the information in the second display region in three different display formats according to each of selection methods of a first selection method in which the attributes assigned to the axis in the row direction are selected, a second selection method in which the attributes assigned to the axis in the column direction are selected, and a third selection method in which a cell that is an intersection between the two kinds of attributes is selected.
